(19) Europäisches Patentamt
European Patent Office
Office européen des brevets

(11) Veröffentlichungsnummer: **0 140 070**
**A1**

# (12) EUROPÄISCHE PATENTANMELDUNG

(21) Anmeldenummer: **84110732.9**

(22) Anmeldetag: **08.09.84**

(51) Int. Cl.[4]: **C 07 D 471/04**
C 07 D 491/20, C 07 D 495/20
C 07 D 217/02, C 07 D 217/04
C 07 D 217/14, C 07 C 131/00
A 61 K 31/47

(30) Priorität: **21.09.83 DE 3333994**

(43) Veröffentlichungstag der Anmeldung:
**08.05.85 Patentblatt 85/19**

(84) Benannte Vertragsstaaten:
**AT BE CH DE FR GB IT LI NL SE**

(71) Anmelder: **Troponwerke GmbH & Co. KG**
**Berliner Strasse 156**
**D-5000 Köln 80(DE)**

(72) Erfinder: **Boltze, Karl-Heinz, Dr.**
**Ackerstrasse 8**
**D-5231 Borod(DE)**

(72) Erfinder: **Davies, Margaret A., Dr.**
**Thebäerstrasse 80**
**D-5000 Köln 30(DE)**

(72) Erfinder: **Junge, Bodo, Dr.**
**Spieckern 23**
**D-5600 Wuppertal 23(DE)**

(72) Erfinder: **Schuurman, Teunis, Dr.**
**Starenweg 17**
**D-5063 Overath(DE)**

(72) Erfinder: **Traber, Jörg, Dr.**
**Löwenburgstrasse 12**
**D-5204 Lohmar 21(DE)**

(74) Vertreter: **Adrian, Albert, Dr. et al,**
**c/o BAYER AG Konzernverwaltung RP Patentabteilung**
**D-5090 Leverkusen 1, Bayerwerk(DE)**

(54) **Neue Pyridoindolderivate, Verfahren zu ihrer Herstellung und ihre Verwendung.**

(57) Die vorliegende Erfindung betrifft neue Pyridoindole und deren Säureadditionssalze, Verfahren zu ihrer Herstellung sowie ihre Verwendung bei der Bekämpfung von Krankheiten, insbesondere ihre Verwendung zur Behandlung von Erkrankungen des zentralen Nervensystems.

EP 0 140 070 A1

TROPONWERKE GmbH & Co. KG 5000 Köln 80
Je/m-c

Neue Pyridoindolderivate, Verfahren zu ihrer Herstellung und ihre Verwendung

Die vorliegende Erfindung betrifft neue Pyridoindole und deren Säureadditionssalze, Verfahren zu ihrer Herstellung sowie ihre Verwendung bei der Bekämpfung von Krankheiten, insbesondere ihre Verwendung zur Behandlung von Erkrankungen des zentralen Nervensystems.

Die Erfindung betrifft Pyridoindole der allgemeinen Formel I

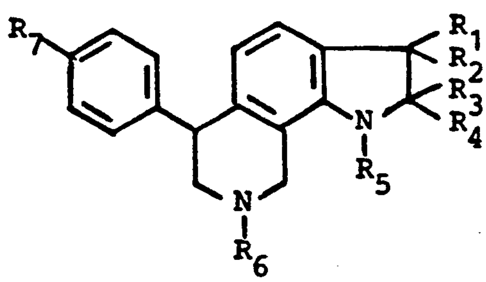

I

in welcher

TP 67-Ausland

$R_1$ für Wasserstoff oder verzweigtes oder geradkettiges $C_1$-$C_4$-Alkyl steht, welches gegebenenfalls durch den Rest 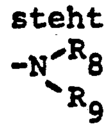 substituiert ist,

$R_2$ und $R_3$ für H stehen oder eine Bindung bilden, oder

$R_1$ und $R_2$ zusammen für Sauerstoff, $-O-CH_2-CH_2-O-$, $-S-CH_2-CH_2-S-$ stehen,

$R_4$ für H oder einen 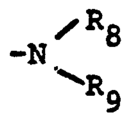 -Rest steht oder

$R_3$ und $R_4$ für Sauerstoff stehen, oder

$R_1$ und $R_4$ Glieder eines N-haltigen Sechsrings sind und

$R_8$ und $R_9$ für H oder $C_1$-$C_4$-Alkyl stehen oder gegebenenfalls mit dem N-Atom einen heterocyclischen 5- oder 6-Ring bilden, der gegebenenfalls auch ein weiteres Heteroatom aus der Reihe N, O oder S enthalten kann,

$R_5$ für H, $C_1$-$C_4$-Alkyl oder die Gruppe 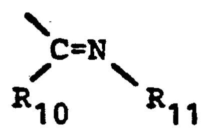 steht, oder

$R_5$ und $R_3$ eine Bindung bilden, und

$R_{10}$ und $R_{11}$ für $C_1$-$C_4$-Alkyl stehen oder Glieder eines N-haltigen 5- oder 6-Rings sind,

$R_6$ für H oder $C_1$-$C_4$-Alkyl steht und

TP 67

$R_7$ für H oder Halogen, insbesondere für F, Cl oder Br steht.

Weiterhin wurde gefunden, daß man die Verbindungen der allgemeinen Formel I erhält, wenn man Verbindungen der allgemeinen Formel II,

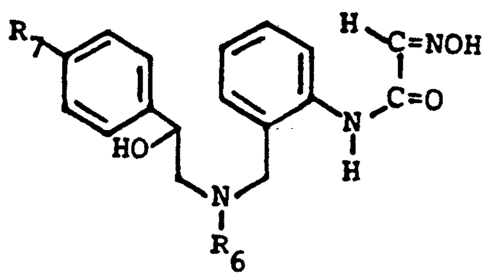

II

in der

$R_6$ und $R_7$ die oben angegebene Bedeutung haben,

einer doppelten Ringschlußreaktion in Gegenwart eines wasserabspaltenden Mittels unterwirft. Man erhält so Isatine der allgemeinen Formel Ia.

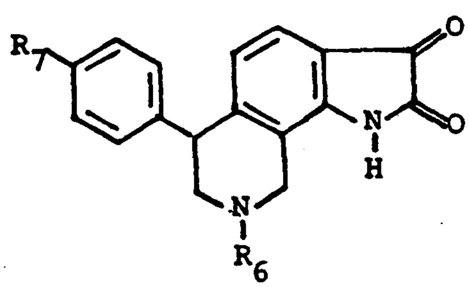

Ia

Verbindungen der Formel Ia werden auch erhalten, wenn man Verbindungen der Formel III,

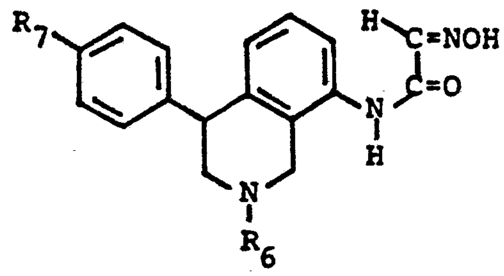

III

in der

$R_6$ und $R_7$ die oben gegebenen Bedeutung besitzen,

in Gegenwart eines wasserabspaltenden Mittels cyclisiert.

Ausgehend von den Isatinen der Formel Ia lassen sich weitere Verbindungen der Formel I herstellen. So erhält man durch Reduktion der Isatine Ia mit komplexen Metallhydriden die Indole Ib,

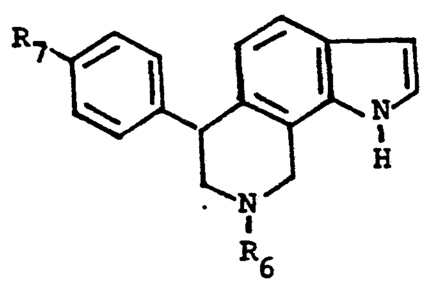

Ib

die ihrerseits weiter zu den Dihydroindolen Ic

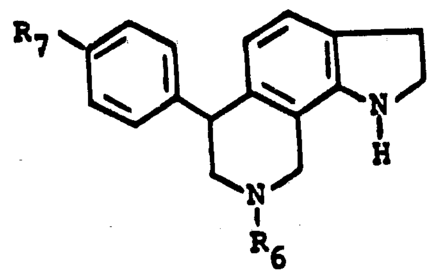

Ic

reduziert werden können. Umsetzung der Indole Ib mit Mannich-Reagenzien (n = 1) oder mit Oxalylchlorid (n = 2), anschließend mit einem primären oder sekundären Amin der Formel IV,

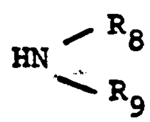

IV

in der

$R_8$ und $R_9$ die oben gegebene Bedeutung besitzen,

mit nachfolgender Reduktion führt zu Verbindungen der Formel Id mit basischen Alkylresten im Indolring.

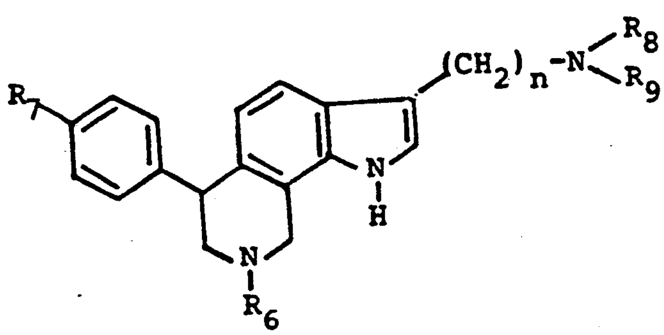

Id

Weitere Umsetzung der Verbindungen der Formel Id (n = 2) mit Formaldehyd liefert ß-Carboline der Formel Ie.

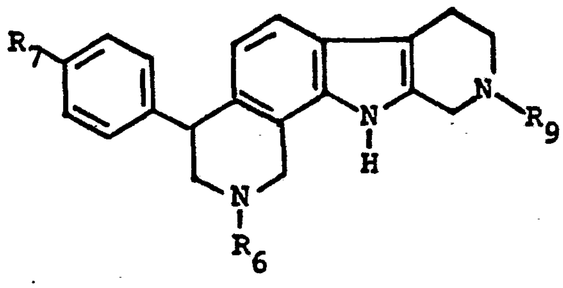

Ie

Verbindungen der Formel If erhält man aus den Isatinen der Formel Ia, wenn man die Ketofunktion mit Ethylenglykol ketalisiert, die Säureamidfunktion mit Lawesson-Reagens schwefelt und dann die Thioamidfunktion mit primären oder sekundären Aminen der Formel IV in eine Amidinfunktion umwandelt.

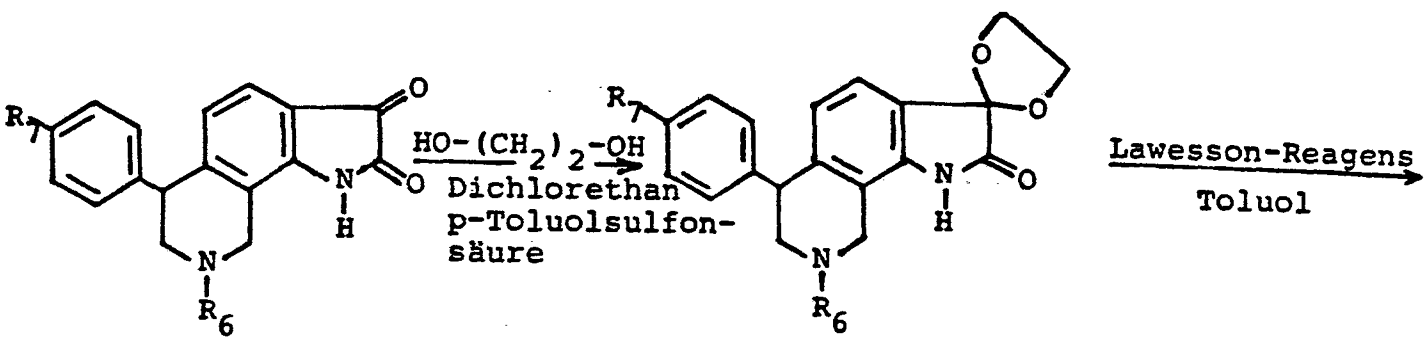


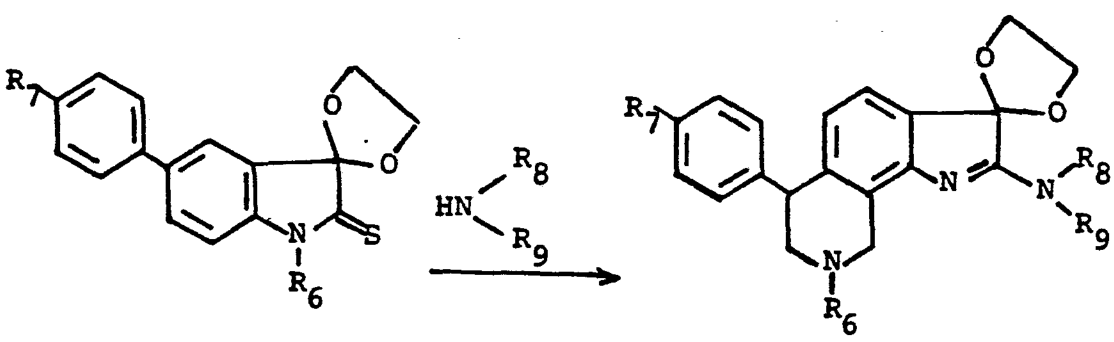

If

Verbindungen der Formel Ig ($R_5$ = $C_1$ - $C_4$-Alkyl)

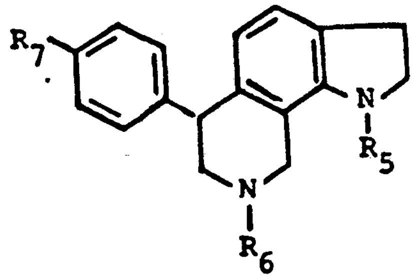

Ig

erhält man aus Verbindungen der Formel Ic durch reduktive Alkylierung mit $C_1$-$C_4$-Aldehyden und $NaCNBH_3$.

Die Umsetzung von Verbindungen der Formel Ic mit Säureamiden der Formel V,

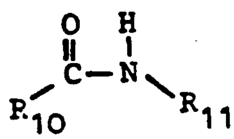

V

in der $R_{10}$ und $R_{11}$ die oben angegebene Bedeutung besitzen, und $POCl_3$ liefert die Verbindungen der Formel Ih.

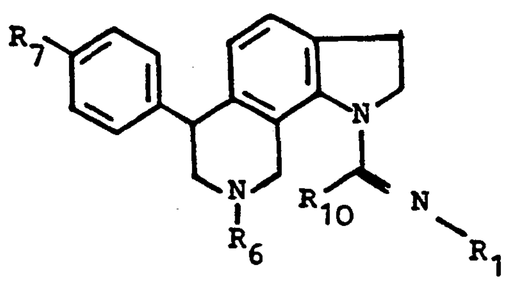

Ih

Die erfindungsgemäßen Wirkstoffe der allgemeinen Formel I zeigen eine ausgeprägte Wirkung auf das zentrale Nervensystem. Sie zeigen anxiolytische, nootrope, insbesondere aber auch antidepressive Wirkungen. Daher eignen sie sich insbesondere zur Behandlung von depressiven Zuständen und stellen somit eine Bereicherung des Arzneimittelschatzes dar.

Bevorzugt sind Verbindungen der Formel I, in denen

$R_1$ für H oder $C_1$-$C_3$-Alkyl steht, das durch den Rest 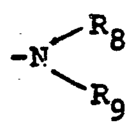 substituiert ist, wobei $R_8$ und $R_9$ unabhängig voneinander für H oder $C_1$-$C_4$-Alkyl stehen oder zusammen mit dem N-Atom einen Piperidin-, Pyrrolidin-, Morpholin- oder Piperazinring bilden,

$R_4$ für H steht,

$R_2$ und $R_3$ für H stehen oder eine Bindung bilden,

$R_5$ für H steht,

$R_6$ für $CH_3$ und

$R_7$ für H oder F steht.

Besonders bevorzugt sind Verbindungen der Formel I, in denen

$R_1$ und $R_4$ für H stehen,

$R_2$ und $R_3$ für H stehen oder eine Bindung bilden,

$R_5$ für H steht,

$R_6$ für $CH_3$ und

$R_7$ für F steht.

Die Herstellung von Verbindungen der Formel Ia wird beispielhaft durch folgendes Formelschema erläutert:

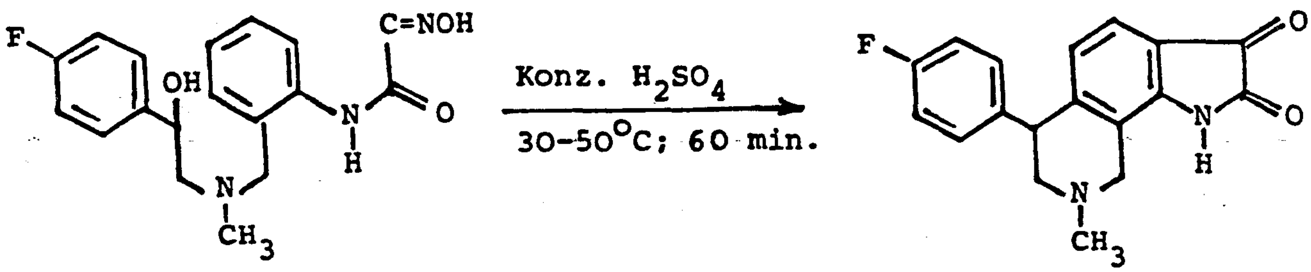


Ein weiteres Verfahren zur Herstellung von Verbindungen der Formel Ia wird durch das folgende Reaktionsschema beispielhaft veranschaulicht:

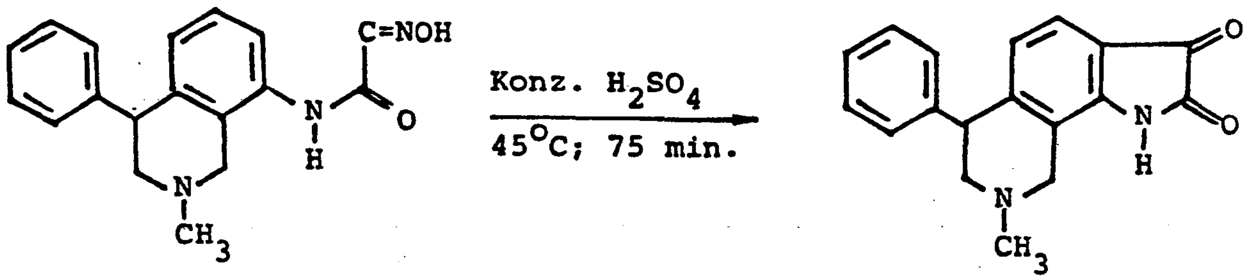


Ia

TP 67

Die beiden folgenden Formelschemata erläutern beispielhaft die Herstellung von Verbindungen der Formeln Ib und Ic:

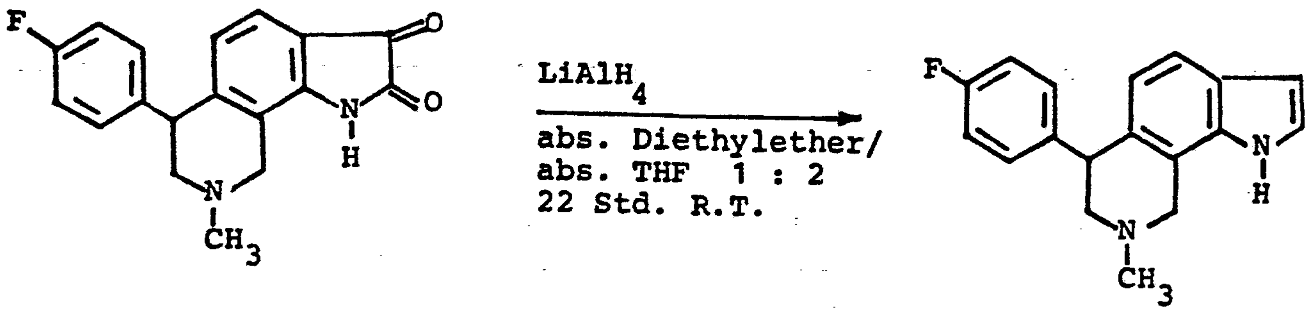


Ib

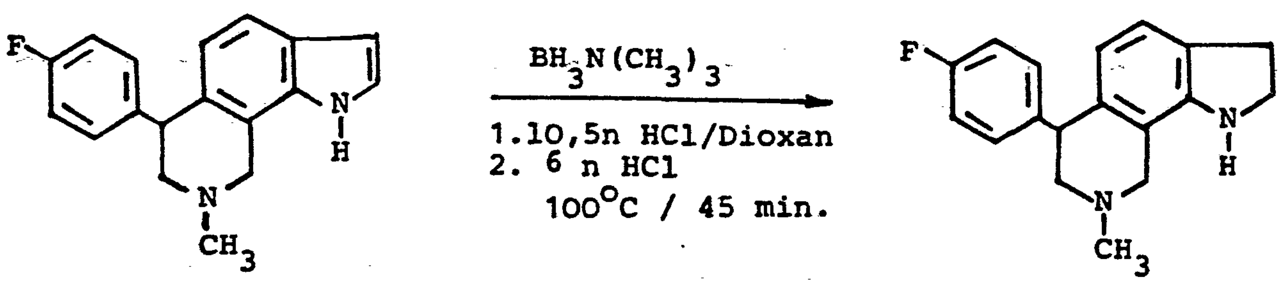


Ic

Ausgehend von den Verbindungen Ic, erhält man die Verbindungen Ig und Ih, wie durch die folgenden Reaktionsgleichungen beispielhaft erläutert wird:

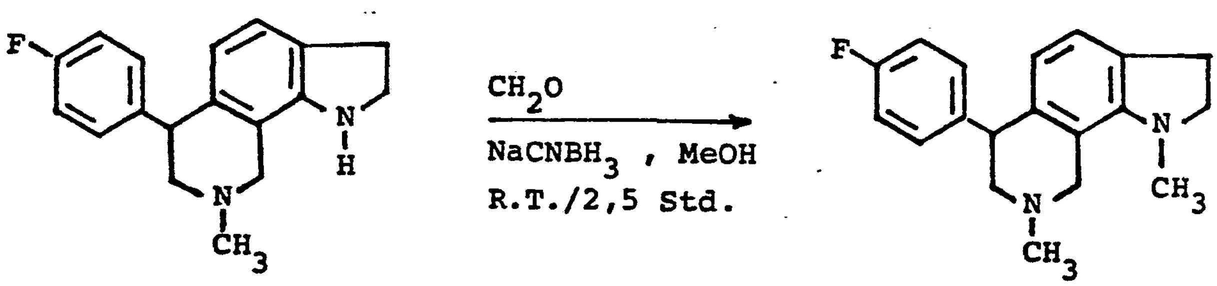

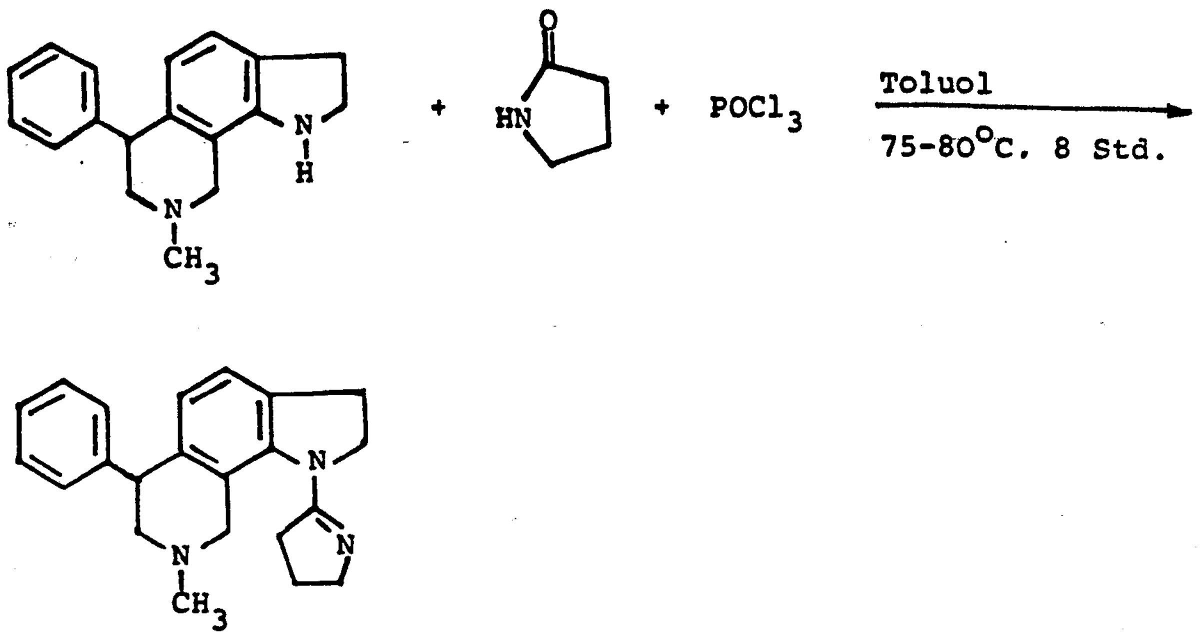


Die Herstellung von Verbindungen der Formel Id veranschaulichen die beiden folgenden Beispiele:

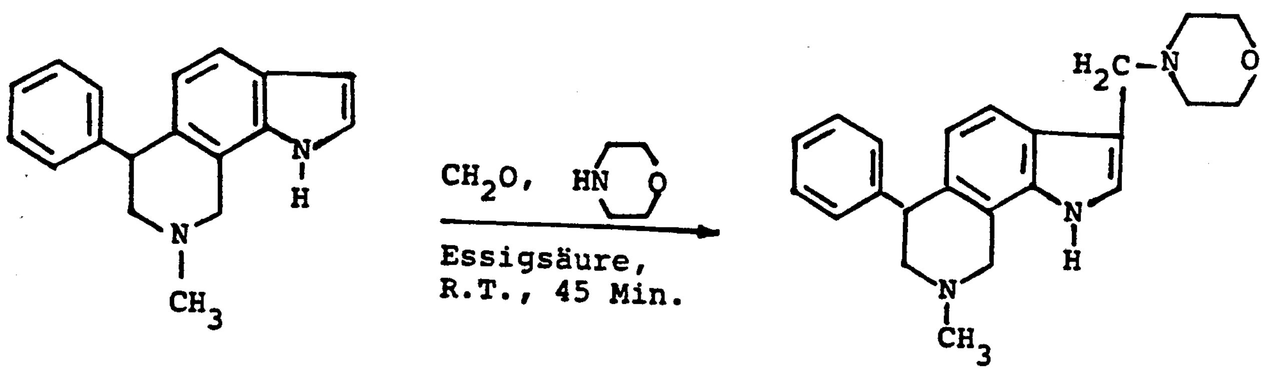


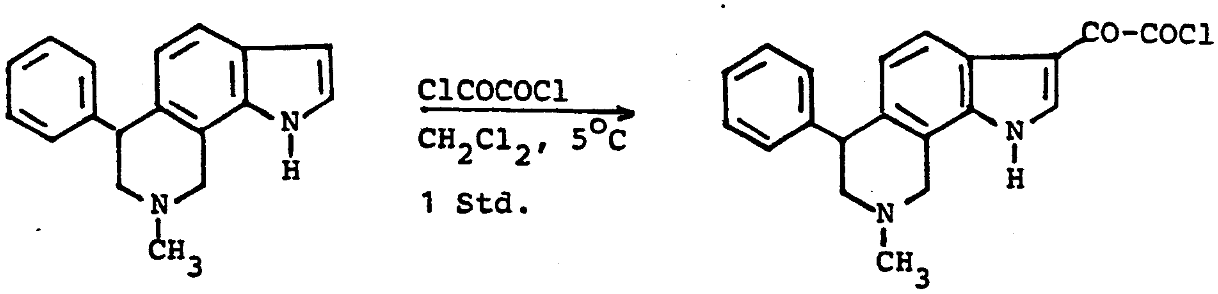

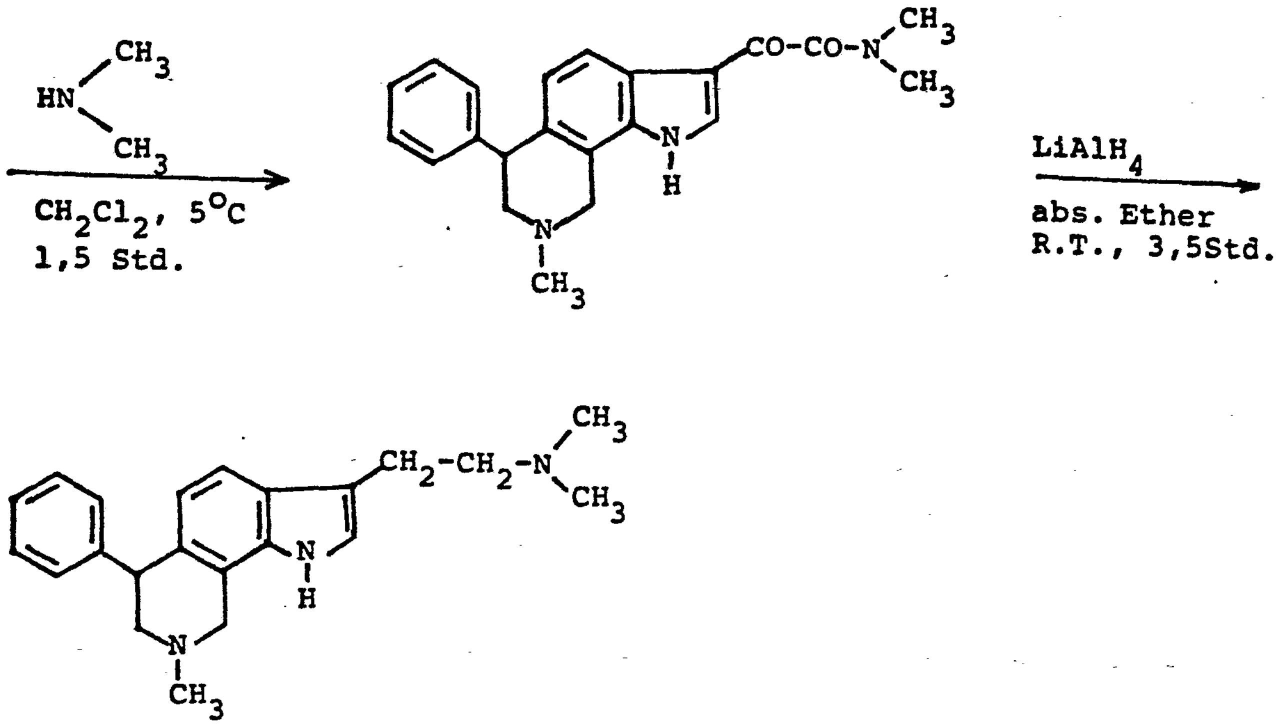


Carboline der Formel Ie erhält man auf dem durch das folgende Beispiel erläuterten Reaktionsweg:

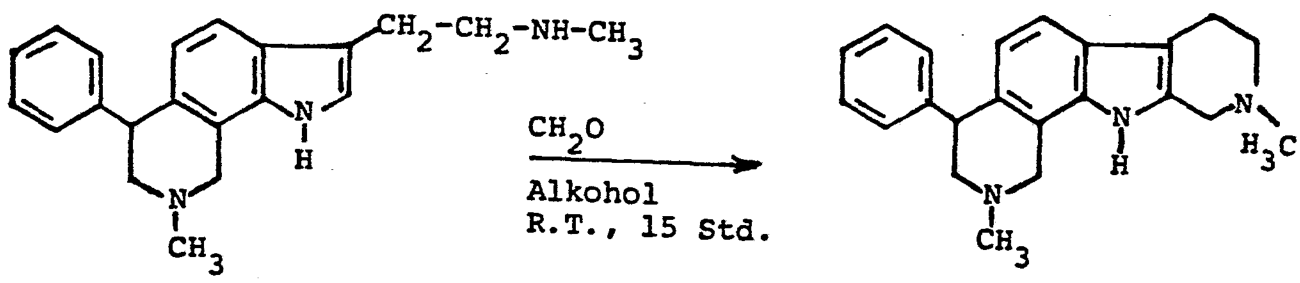


Das folgende Schema beschreibt beispielhaft die Herstellung von Amidinen der Formel If:

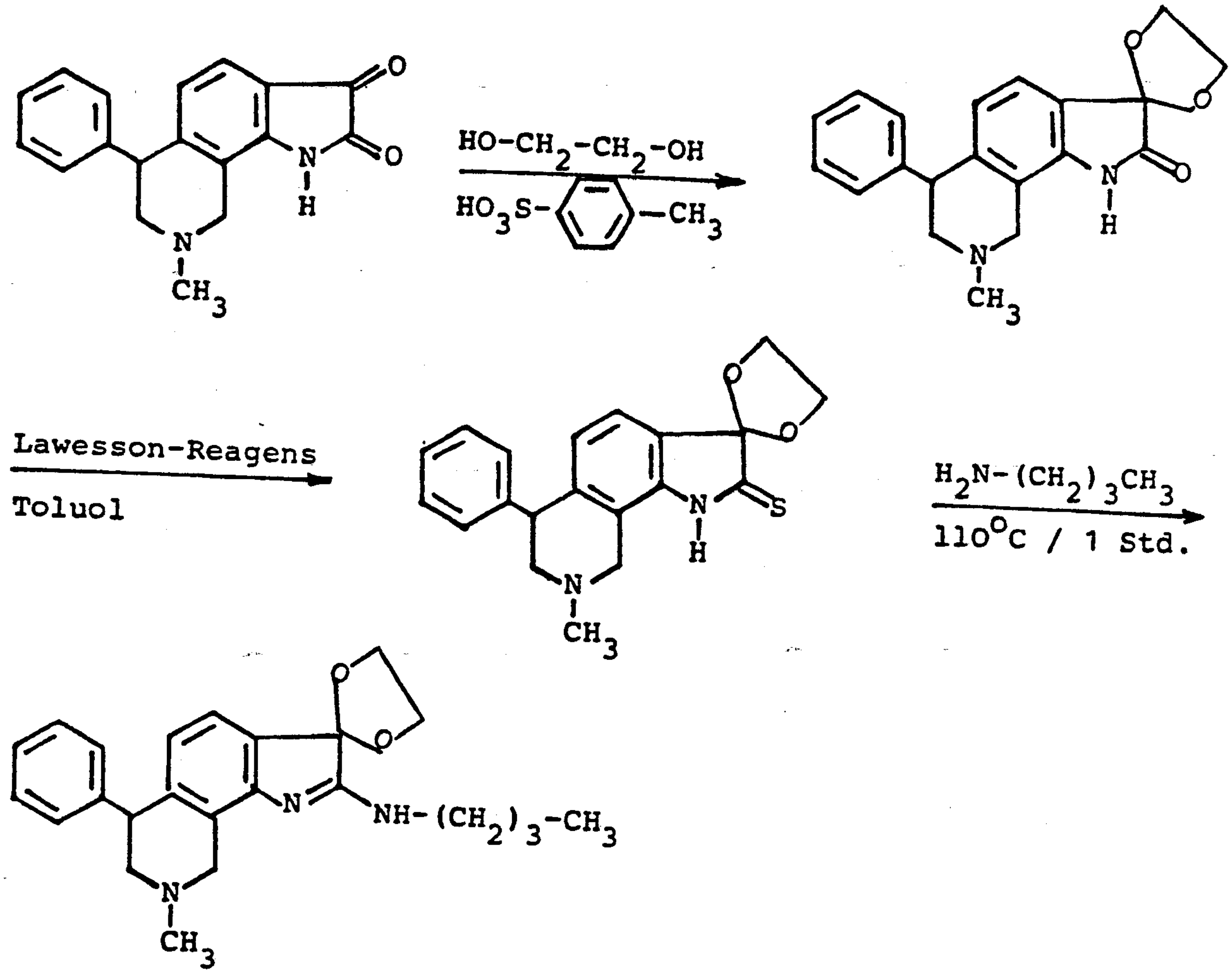
HO-CH2-CH2-OH
HO3S-
-CH3
Lawesson-Reagens
Toluol
H2N-(CH2)3CH3
110°C / 1 Std.

Die Umsetzung der Oxime II zu den Isatinen Ia wird in einer starken wasserabspaltenden Säure als Lösungsmittel durchgeführt. Als solche eignen sich konzentrierte Schwefelsäure oder auch Polyphosphorsäure. Die Reaktion kann bei Temperaturen zwischen ca. 10°C und 120°C durchgeführt werden. Bevorzugt wird die Reaktion zwischen 30°C und 80°C, inbesondere zwischen 40°C und 50°C durchgeführt. Die Reaktionszeiten bewegen sich je nach Temperatur zwischen wenigen Minuten und einigen Stunden. Wird die Reaktion zwischen 40°C und 50°C durchgeführt, so beträgt die Reaktionszeit ca. 1 Stunde.

Unter den gleichen Reaktionsbedingungen erfolgt auch die Umsetzung der Oxime III zu den Isatinen Ia.

Die Reduktion der Isatine Ia zu den Indolen Ib wird vorzugsweise mit komplexen Metallhydriden, insbesondere mit $LiAlH_4$, durchgeführt. Als Lösungsmittel finden inerte aprotische Lösungsmittel Verwendung, insbesondere Ether, wie Diethylether, Tetrahydrofuran, Dioxan, 1,2-Dimethoxyethan, Diglyme oder Gemische dieser Lösungsmittel. Die Reaktion wird bei Temperaturen zwischen Raumtemperatur und der Siedetemperatur des Lösungsmittels, insbesondere bei Raumtemperatur durchgeführt.

Die Reduktion der Indole Ib zu den Dihydroindolen Ic wird vorzugsweise mit Borwasserstoffverbindungen durchgeführt. Bevorzugt verwendet man Aminoboran-Komplexe oder auch $NaBH_4$ oder $NaCNBH_3$ in organischen Säuren, wie Essigsäure oder Trifluoressigsäure. Als Lösungsmittel

werden Gemische von wäßrigen Mineralsäuren, beispielsweise wäßrige Salzsäure, mit organischen Lösungsmitteln, wie Dioxan, Tetrahydrofuran oder Ethanol, oder aber organische Carbonsäuren, wie Essigsäure oder Trifluoressigsäure, verwendet. Die Reaktion wird bei Temperaturen zwischen Raumtemperatur und der Siedetemperatur des Lösungsmittels durchgeführt. Für die Herstellung der Mannich-Basen Id (n = 1) aus den Indolen Ib verwendet man als Lösungsmittel Gemische von organischen Lösungsmitteln mit wäßrigen Mineralsäuren oder organischen Säuren oder auch wäßrige organische Carbonsäuren. Bevorzugt wird die Reaktion in wäßriger Essigsäure durchgeführt. Die Reaktion wird zwischen 0°C und 100°C, bevorzugt bei Raumtemperatur durchgeführt.

Die Umsetzung der Indole Ib mit Oxalylchlorid zur Herstellung der Verbindungen Id (n = 2) erfolgt in einem inerten organischen Lösungsmittel in Gegenwart eines säurebindenden Mittels.

Als Lösungsmittel seien vorzugsweise genannt: Dioxan, Tetrahydrofuran, Toluol, Chlorbenzol, Methylenchlorid, Dichlorethan. Als Säurebinder werden anorganische Salze, wie Kaliumkarbonat oder Natriumhydrogenkarbonat oder tertiäre organische Basen, wie Triethylamin oder DBU, verwendet. Besonders bevorzugt ist die Verwendung von Dioxan als Lösungsmittel und $K_2CO_3$ als säurebindendem Mittel.

Die Reaktion wird bei Temperaturen zwischen -20°C und 60°C, bevorzugt um 0°C durchgeführt. Die weitere Umsetzung der so erhaltenen Zwischenprodukte mit Aminen der Formel IV erfolgt unter den gleichen Reaktionsbedingungen wie oben angegeben, bevorzugt in Dioxan als Lösungsmittel und mit $K_2CO_3$ als Hilfsbase.

Die Reduktion dieser Zwischenprodukte zu Verbindungen der Formel Id (n = 2) wird vorzugsweise mit komplexen Metallhydriden, insbesondere mit $LiAlH_4$, durchgeführt. Als Lösungsmittel finden inerte aprotische Lösungsmittel, insbesondere Ether, wie Diethylether, Tetrahydrofuran, Dioxan, 1,2-Dimethoxyethan, Diglyme oder Gemische dieser Lösungsmittel Verwendung. Besonders bevorzugt ist die Verwendung von absolutem THF als Lösungsmittel. Die Reaktion wird bei Temperaturen zwischen Raumtemperatur und der Siedetemperatur des Lösungsmittels durchgeführt, besonders bevorzugt bei der Siedetemperatur des Lösungsmittels.

Die Umsetzung von Verbindungen der Formel Id (n = 2, $R_8$ = H) mit Formaldehyd zu ß-Carbolinen der Formel Ie wird vorzugsweise in wäßrigen Mineralsäuren durchgeführt; besonders bevorzugt ist die Verwendung wäßriger Salzsäure. Die Reaktion erfolgt zwischen 0° und 80°C, bevorzugt bei Raumtemperatur.

Die Umsetzung der Isatine Ia mit Ethylenglykol zur Herstellung der Verbindungen If erfolgt in einem inerten organischen Lösungsmittel durch Kochen am Wasserabscheider in Gegenwart eines sauren Katalysators. Als Lösungsmittel seien genannt: Dichlorethan, Toluol, Chlorbenzol. Bevorzugt ist die Verwendung von Dichlorethan als Lösungsmittel und p-Toluolsulfonsäure als Katalysator.

Die Schwefelung der so erhaltenen Zwischenprodukte wird mit Lawesson-Reagens

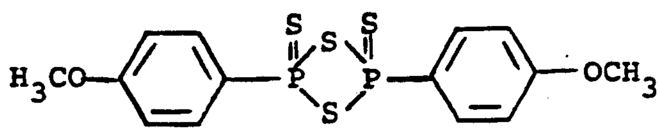

oder $P_2S_5$ in einem inerten organischen Lösungsmittel durchgeführt. Als solche finden Toluol, Xylol, Dimethoxyethan, THF oder Dioxan Verwendung. Besonders bevorzugt ist die Verwendung von Toluol als Lösungsmittel. Bevorzugt wird die Reaktion bei der Siedetemperatur des Lösungsmittels durchgeführt.

Die Umsetzung der so erhaltenen Thioamide mit Aminen der Formel IV zu den Amidinen der Formel If kann mit oder ohne Lösungsmittel erfolgen. Bevorzugt ist die Verwendung eines Überschusses des Amins der Formel IV als Lösungsmittel. Die Reaktion wird bei Temperaturen zwischen 50°C und 150°C durchgeführt, bevorzugt bei Temperaturen zwischen 80°C und 120°C.

Die Herstellung von Verbindungen der Formel Ig ($R_5$ = $C_1$-$C_4$-Alkyl) erfolgt durch Alkylierung der Amine Ic. Bevorzugt ist die reduktive Alkylierung der Amine Ic mit

Aldehyden und Ketonen in Gegenwart eines Wasserstoffdonor. Als Lösungsmittel verwendet man polare organische Lösungsmittel oder deren Gemische mit Wasser. Bevorzugt ist die Verwendung von Alkoholen als Lösungsmittel, insbesondere die Verwendung von Methanol. Die Reaktion wird in einem pH-Bereich zwischen 3 und 8 durchgeführt, bevorzugt in einem pH-Bereich zwischen 5 und 6. Bevorzugt wird dieser pH-Wert durch Zugabe einer organischen Säure, insbesondere durch Zugabe von Essigsäure eingestellt. Die Reaktion wird bei Temperaturen zwischen 0°C und der Siedetemperatur des Lösungsmittels durchgeführt; bevorzugt wird die Reaktion bei Raumtemperatur durchgeführt.

Die Umsetzung der Amine Ic mit Säureamiden der Formel V in Gegenwart von $POCl_3$ zu Amidinen der Formel Ih erfolgt in inerten organischen Lösungsmitteln. Genannt seien Toluol, Xylol, Dichlorethan, Chloroform, Tetrahydrofuran und Dioxan. Bevorzugt ist die Verwendung von Toluol. Die Umsetzung des Säureamids mit $POCl_3$ erfolgt bei Temperaturen zwischen 0° und 80°C. Bevorzugt wird die Umsetzung bei Raumtemperatur durchgeführt. Die Umsetzung des Amins mit dem aktivierten Säureamid wird bei Temperaturen zwischen Raumtemperatur und der Siedetemperatur des Lösungsmittels durchgeführt. Bevorzugt wird die Reaktion zwischen 70°C und 90°C durchgeführt.

Die Ausgangsstoffe der Formel II sind neu. Sie lassen sich aus den Aminen der Formel VI

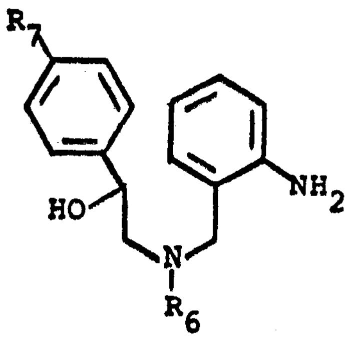

VI

durch Umsetzung mit Chloralhydrat und Hydroxylamin herstellen. (Isatin-Synthese nach Sandmeyer (Krauch, Kunz; Reaktionen der organischen Chemie, Dr. Alfred Hüthig, Verlag Heidelberg, 5. Auflage).)

Die Amine der Formel VI sind zum Teil bekannt. Soweit sie neu sind, lassen sie sich nach literaturbekannten Verfahren, wie sie in der Deutschen Patentanmeldung - DE-PS 1 670 694 - beschrieben sind, herstellen.

Die Ausgangsstoffe der Formel III sind neu. Sie lassen sich aus Aminen der Formel VII

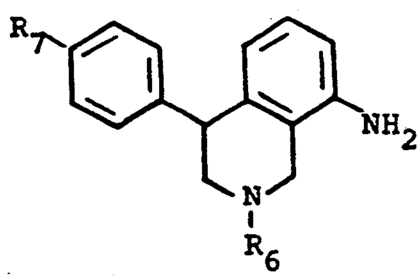

VII

herstellen (Isatin-Synthese nach Sandmeyer).

Die Amine der Formel VII sind zum Teil aus der deutschen Patentanmeldung - DE-PS 1 670 694 - bekannt. Neue Verbindungen der Formel VII lassen sich in Analogie zu den dort beschriebenen Verbindungen herstellen.

Zur vorliegenden Erfindung gehören pharmazeutische Zubereitungen, die neben nicht-toxischen, inerten pharmazeutisch geeigneten Trägerstoffen eine oder mehrere Verbindungen der obigen allgemeinen Formel I enthalten oder die aus einer oder mehreren Verbindungen der obigen Formel bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen, vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben Tagesdosis oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshiflsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum-, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe, wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylenglykol, Suspendiermittel, z.B. ethoxylierte Isostearylalkohole, Polyoxyethylensorbit- und sorbitanester, mikrokristalline

Cellulose, Aluminiummethanhydroxid, Bentonit, Agar-Agar und Traganth oder Gemische dieser Stoffe enthalten.

Die genannten Formulierungsformen können auch Färbemittel, Konservierungsstoffe sowie geruchs- und geschmachsverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer Verbindungen der obigen Formel auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehören auch die Verwendung der Verbindungen der obigen Formel sowie die Verwendung von pharmazeutischen Zubereitungen, die eine oder mehrere Verbindungen der oben angegebenen Formel enthalten, in der Human- und Veterinärmedizin zur Verhütung, Besserung und/oder Heilung, insbesondere der oben angeführten Erkrankungen.

<u>TP 67</u>

Die Wirkstoffe oder die pharmazeutischen Zubereitungen können oral, parenteral, intraperitoneal und/oder rektal, vorzugsweise oral, gegebenenfalls auch in einer magensaftresistenten Zubereitung appliziert werden.

Im allgemeinen hat es sich als vorteilhaft erwiesen, den oder die Wirkstoffe in Mengen von etwa 0,01 bis etwa 100, vorzugsweise 0,1 bis 10 mg/kg Körpergewicht je 24 Stunden, verteilt auf 1 bis 6 Verabreichungen und zwar vor oder/und während oder/und nach der Mahlzeit oral zu applizieren. Eine Einzelgabe enthält den oder die Wirkstoffe, vorzugsweise in Mengen von etwa 0,1 bis etwa 5 mg/kg Körpergewicht. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Nachstehend werden einige Testergebnisse der neuen Verbindungen der allgemeinen Formel I angegeben, die auf anxiolytische, nootrope und antidepressive Eigenschaften geprüft wurden. Als Leittest für anxiolytische Wirkung diente der Tedeschi-Test (Tedeschi; et al., J. Pharmacol. Exp.Ther. 129, 28-34, 1954), für die antidepressive Wirkung der Antitetrabenazin-Test (J.L. Howard; et al., in: Antidepressants: Neurochemical, Behavioral and Clinical Perspectives, herausgegeben von S.J. Enna et al., Raven Press N.Y., S. 107-120, 1981) und Amphetaminpotenzierungstest (J.L. Howard; et al., in: Antidepressants: Neurochemical, Behavioral and Clinical Perspectives, herausgegeben von S.J. Enna et al., Raven Press N.Y., S. 107-120, 1981). Beispielsweise wurden für die antidepressiven Wirkungen der erfindungsgemäßen Substanzen folgende Werte erhalten:

1) Tetrabenazin Antagonismus

Antidepressiv wirksame Substanzen antagonisieren die durch Tetrabenazin induzierte Ptosis bei Mäusen. Der $DE_{50}$-Wert gibt diejenige Dosis an, bei der die durch 20 mg/kg i.p. Tetrabenazin induzierte Ptosis zu 50 % reduziert ist. Dazu seien Beispiele genannt:

| Verbindung | $DE_{50}$ (mg/kg i.p.) |
|---|---|
| 1 a (als freie Base) | 1,0 |
| 1 b (als freie Base) | 2,0 |
| 2 a (als Maleinat Salz) | 0,3 |
| 2 b (als Maleinat Salz) | 0,2 |
| 3 a (als Hydrochlorid) | 0,1 |
| 3 b (als Hydrochlorid) | 0,01 |
| 4 (als Hydrochlorid) | 0,01 |
| 7 a (als Hydrochlorid) | 0,1 |
| 7 b (als Hydrochlorid) | 0,1 |
| 6 c (als Hydrochlorid) | 2,0 |

2) Amphetamin-Potenzierung

Antidepressiv wirksame Substanzen potenzieren das Amphetamin-induzierte stereotype Verhalten bei der Ratte.

Der angegebene $DE_{50}$-Wert ist die Dosis, bei der das Amphetamin-induzierte Verhalten nach Gabe von 2 mg/kg DL-Ampethaminsulfat i.v. um 50 % verstärkt ist. Dazu seien Beispiele genannt:

| Verbindung | $DE_{50}$ (mg/kg i.p.) |
|---|---|
| 2 a (als Maleinat Salz) | 2,4 |
| 2 b (als Maleinat Salz) | 3,9 |
| 3 a (als Hydrochlorid) | 1,2 |
| 3 b (als Hydrochlorid) | 2,2 |

Die vorliegende Erfindung soll durch die nachfolgenden Beispiele noch näher erläutert werden.

Beispiel 1

8-Methyl-6-(4-fluorphenyl)-6,7,8,9-tetrahydro-1H-pyrido-[4,3-g]indol-2,3-dion

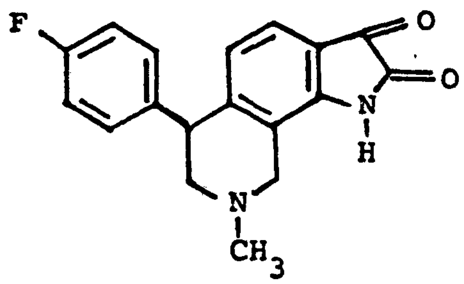

1 a

Zu 78 ml (1,37 Mol) konz. $H_2SO_4$, die auf 35°C erwärmt ist, gibt man unter Rühren und Feuchtigkeitsausschluß tropfenweise eine Lösung von 27 g (0.0781 Mol) 2-Methyl-(2-(hydroxiiminoacetylamino)benzyl)amino-1-(4-fluorphenyl)ethanol in 35 ml $CH_2Cl_2$ und rührt anschließend bei 45°C 1 Std. nach. Die Reaktionslösung wird auf 500 g Eis gegossen und bei 0°C mit 25 %iger NaOH auf pH-6.8 eingestellt. Den Niederschlag saugt man ab, wäscht mit $H_2O$ und trocknet im Exsikkator bei 60°C über $P_2O_5$ unter Wasserstrahlvakuum.

Ausbeute: 22,1 g (91 %) gelbgefärbte Substanz;
Fp (Micro-Kofler): 197° - 200°C unter Zersetzung

Analog wurde hergestellt:

8-Methyl-6-phenyl-6,7,8,9-tetrahydro-1H-pyrido/4,3-g/indol-2,3-dion

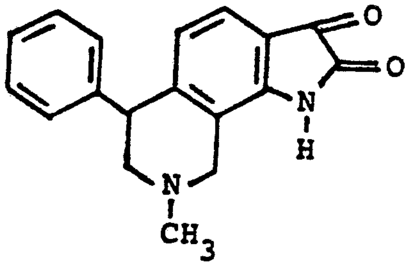

1 b

Ausbeute: 90 % d.Th.; Fp (Mettler FP61): 214.7°C

Herstellung der Ausgangsstoffe:

2-Methyl(2-(hydroxiiminoacetylamino)benzyl)amino)-1-phenylethanol

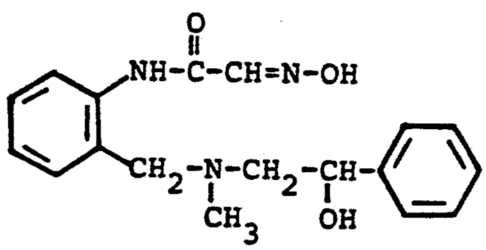

189.9 g (1.146 Mol) Chloral-Hydrat werden unter Rühren in 2.6 l $H_2O$ gelöst und bei Raumtemperatur mit einer Lösung von 306 g 2-Methyl(2-aminobenzyl)amino-1-phenylethanol in 666 ml $H_2O$ versetzt. Nach 5 Min. werden 1280 g $Na_2SO_4$ und eine Lösung von 229 g (3.297 Mol) Hydroxylamin-Hydrochlorid in 1.08 l $H_2O$ zugegeben. Anschließend erwärmt man innerhalb 40 Min. auf 106°C Innentemperatur. Die Reaktionstemperatur wird 2 Min. auf

106°C gehalten und dann durch rasches Abkühlen mit Eis/ $H_2O$ auf Raumtemperatur gebracht. Die Reaktionsmischung wird mit MeOH versetzt bis die organischen Substanzen gelöst sind. Mit 25 %iger NaOH stellt man einen pH-Wert von 7.8 ein und zieht am Rotationsverdampfer das Lösungsmittel ab. Die wäßrige Phase wird mit Essigsäureethylester extrahiert, die organische Phase über $Na_2SO_4$ getrocknet und das Lösungsmittel abrotiert. Den Rückstand befreit man im Ölpumpenvakuum von Lösungsmittelresten und trocknet anschließend im Exsikkator über $P_2O_5$.

Ausbeute: 317 g (91 %)

Auf analogem Wege wurde erhalten:

2-(Methyl-(2-(hydroxiiminoacetylamino)benzyl)amino)-1-(4-fluorphenyl)ethanol

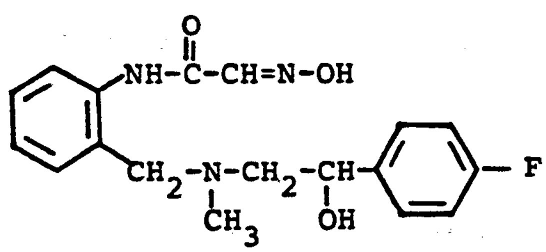

Das Rohprodukt wurde an Kieselgel 60 mit Essigsäureethylester als Fließmittel chromatografiert.

Ausbeute: 76 % d.Th.

TP 67

2-(Methyl-(2-aminobenzyl)amino)-1-(4-fluorphenyl)ethanol

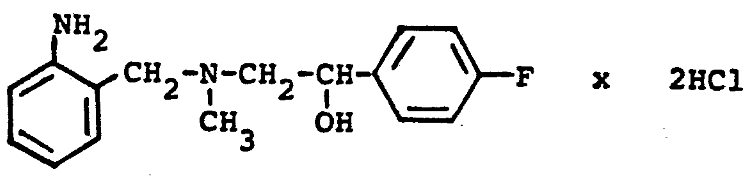

Eine Lösung von 124.5 g (0.409 Mol) 2-Methyl-(2-nitrobenzyl)-amino-1-(4-fluorphenyl)ethanol) in 150 ml Ethanol und 500 ml Essigsäureethylester wird mit 1 g $PtO_2$ bei Raumtemperatur und Atmosphärendruck bis zur Aufnahme von 3 Mol-Äquivalent $H_2$ hydriert. Man filtriert den Katatlysator ab, dampft das Filtrat am Rotationsverdampfer ein und löst den Rückstand in 450 ml $CH_2Cl_2$. Die $CH_2Cl_2$-Phase wird 3 mal mit je 200 ml $H_2O$ gewaschen, über $Na_2SO_4$ getrocknet. Nach Filtration wird mit HCl/Diethylether versetzt. Das ausgefallene Salz wird abgesaugt, mit Diethylether gewaschen und im Exsikkator über NaOH bei 60°C unter Wasserstrahlvakuum getrocknet.

Ausbeute: 136 g hygroskopisches Hydrochlorid; Fp (Micro-Kofler): 125° - 127°C.

2-(Methyl(2-nitrobenzyl)amino)-1-(4-fluorphenyl)ethanol

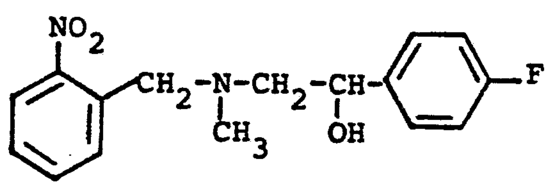

TP 67

140 g (0.413 Mol) 2-Methyl-(2-nitrobenzyl)amino-1-(4-fluorphenyl)ethanon werden in 82.8 ml MeOH gelöst, auf 4°C abgekühlt und unter Rühren tropfenweise innerhalb 2 Stdn. mit einer Lösung von 37.83 g (0.657 Mol) Natriumborhydrid in 270 ml 0.1 n NaOH versetzt. Das Reaktionsgemisch wird 1 Std. bei Raumtemperatur gerührt. Man engt am Rotationsverdampfer ein und extrahiert mit Essigsäureethylester. Die organische Phase trocknet man über $Na_2SO_4$ und zieht das Lösungsmittel ab. Der ölige Rückstand wird im Ölpumpenvakuum von Lösungsmittelresten befreit.

Ausbeute: 124.5 g (99 %).

<u>2-(Methyl(2-nitrobenzyl)amino)-1-(4-fluorphenyl)ethanon</u>

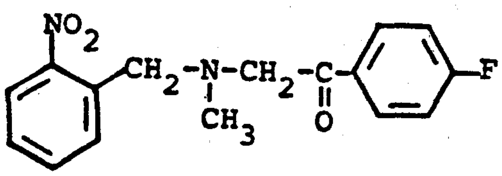 x HCl

135.8 g (0.67 Mol) Methyl-(2-nitrobenzyl)amin und 187.5 ml (1.339 Mol) Triethylamin werden unter Rühren bei Raumtemperatur in 1.87 l Ethanol gelöst und anschließend portionsweise mit 148 g (0.682 Mol) 2-Brom-1-(4-fluorphenyl)ethanon versetzt. Die Reaktionsmischung wird 3 Std. gerührt. Dann zieht man das Lösungsmittel am Rotationsverdampfer ab und löst den Rückstand in 1.3 l Toluol. Die Toluol-Phase wäscht man mehrmals mit $H_2O$, trocknet über

$Na_2SO_4$ und versetzt mit HCl/Diethylether. Der schmierige Niederschlag wird durch Dekantieren vom Toluol getrennt und mit Aceton zur Kristallisation gebracht. Der farblose Niederschlag wird abgesaugt und getrocknet.

Ausbeute: 158.5 g (70 %); Fp (Mettler FP61): 171.9°C.

8-Methyl-6-phenyl-6,7,8,9-tetrahydro-1H-pyrido[4,3-g]-indol 2,3-dion

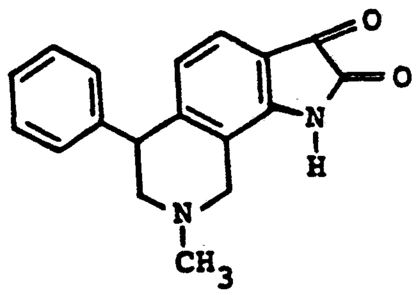

1 b

17,5 g (0,0566 Mol) N-Methyl-4-phenyl-8-(hydroxyimino-acetylamino)isochinolin wurden in kleinen Portionen zu 43,75 ml konz. $H_2SO_4$ bei 45°C gegeben, und das Gemisch wurde 75 Min. auf 45°C erwärmt. Es wurde auf Raumtemperatur gekühlt und auf 450 g Eis gegeben. Dann wurde mit 2 n NaOH ein pH von 6,8 eingestellt. Der ausgefallene Niederschlag wurde abgesaugt und mit Wasser gewaschen. Nach Trocknen erhält man 15,3 g (92,5% d.Th.) orange gefärbtes Rohprodukt. (Fp des Reinprodukts = ca. 188°C Zers.).

Herstellung der Ausgangsverbindung:

N-Methyl-4-phenyl-8(hydroxiiminoacetylamino)isochinolin

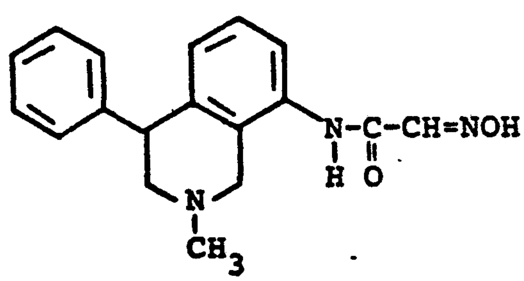

0140070

Zu 6,23 g (0,0376 Mol) Chloralhydrat in 86 ml Wasser wurde eine Lösung von 10,75 g (0,0345 Mol) N-Methyl-4-phenyl-8-amino-isochinolinhydrochlorid in 22 ml $H_2O$ gegeben. Nach 5 Minuten wurden 42,5 g $Na_2SO_4$ und eine Lösung von 7,6 g (0,109 Mol) Hydroxylaminhydrochlorid in 36 ml $H_2O$ hinzugefügt. Das Reaktionsgemisch wurde innerhalb von 40 Minuten auf 106°C (Innentemperatur) erwärmt. Nach 2 Minuten wurde mit Eis/$H_2O$-Kühlung rasch auf 18°C abgekühlt. Das Reaktionsgemisch wurde mit 200 ml Methanol versetzt und mit 2 n NaOH ein pH von 7,6 eingestellt. Der ausgefallene Niederschlag wurde abgesaugt und gründlich mit $H_2O$ gewaschen. Nach Trocknen wurden 9,7 g (90,2% d.Th.) Rohprodukt vom Fp. 151,3°C (Mettler FP 61) erhalten.

Beispiel 2:

8-Methyl-6-phenyl-6,7,8,9-tetrahydro-1H-pyrido/4,3-g/-indol

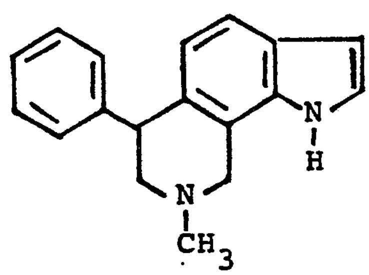

2 a

20 g (0.524 Mol) $LiAlH_4$ werden unter Rühren, Stickstoffspülung und Feuchtigkeitsausschluß bei Raumtemperatur in 400 ml abs. Diethylether aufgeschlämmt. Anschließend werden 550 ml abs. THF zugegeben und dann portionsweise

0140070

34 g (0.116 Mol) 8-Methyl-6-phenyl-6,7,8,9-tetrahydro-1H-pyrido /4,3-g7indol-2,3-dion. Das Reaktionsgemisch wird 3 1/2 Std. bei Raumtemperatur nachgerührt. Zur Zersetzung des Reaktionskomplexes tropft man eine Lösung von 42 ml $H_2O$ in 160 ml THF unter Kühlung zu. Der anorganische Feststoff wird abfiltriert und das Filtrat am Rotationsverdampfer vom Lösungsmittel befreit. Den Rückstand chromatografiert man an Kieselgel 60 mit einem Laufmittelgemisch: Essigsäureethylester und 8.3 % MeOH. Die Reinsubstanz wird im Exsikkator über $P_2O_5$ bei 60°C unter Wasserstrahlvakuum getrocknet.

Ausbeute: 9.1 g (30%); Fp (Mettler FP61): 157.9°C.

Analog wurde hergestellt:

8-Methyl-6-(4-fluorphenyl)-6,7,8,9-tetrahydro-1H-pyrido-/4,3-g7indol

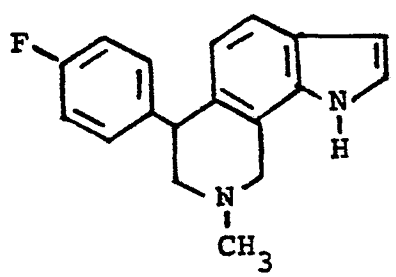

2 b

Ausbeute: 44 % d. Th.; Fp (Mettler FP 61): 205.5 °C.

TP 67

Herstellung eines Salzes mit Maleinsäure

2.0 g (0.00713 Mol) 8-Methyl-6-(4-fluorphenyl)-6,7,8,9-tetrahydro-1H-pyrido/4,3-g/indol werden in 10 ml Ethanol aufgeschlämmt und unter Rühren mit einer Lösung von 0.91 g (0.00784 Mol) Maleinsäure in 10 ml Ethanol versetzt. Die Reaktionsmischung wird 1 Std. bei Raumtemperatur aufbewahrt. Das kristalline Produkt saugt man ab, wäscht mit Ethanol/Ether und trocknet im Exsikkator bei 95°C über $P_2O_5$ unter Wasserstrahlvakuum.

Ausbeute: 2.4 g (85 %); Fp (Mettler FP 61): 170.5°C.

Beispiel 3

8-Methyl-6-phenyl-2,3,6,7,8,9-hexahydro-1H-pyrido/4,3-g/indol

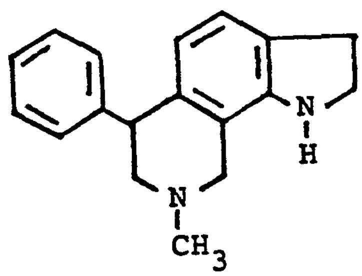

3 a

Zu einem Gemisch von 26 g (0.1 Mol) 8-Methyl-6-phenyl-6,7,8,9-tetrahydro-1H-pyrido/4,3-g/indol und 29.2 g (0.4 Mol) Trimethylaminoboran in 150 ml abs. Dioxan wird unter Rühren und Feuchtigkeitsausschluß bei einer Temperatur von 16°C tropfenweise 12,5 ml (0.131 Mol) 10.5 n Salzsäure zugesetzt. Anschließend erhitzt man 30 Min. unter Rückfluß, kühlt auf Raumtemperatur ab, fügt 50 ml (0.3 Mol) 6 n HCl hinzu und erhitzt noch einmal 15 Min. unter Rückfluß. Nach Abkühlung auf Raumtemperatur wird das Reaktionsgemisch mit 600 ml $H_2O$ versetzt, über Kieselgur filtriert und das Filtrat nach Zugabe weiterer 600 ml $H_2O$ mit 20 % NaOH auf pH 9 eingestellt. Nun wird die wäßrige Phase mehrmals mit $CH_2Cl_2$ extrahiert und nach Trocknung über $Na_2SO_4$ das Lösungsmittel am Rotationsverdampfer abdestilliert. Den öligen Rückstand chromatografiert man an Kieselgel (Kieselgel 60 der Fa. E. Merck, Darmstadt) mit Essigsäureethylester und MeOH (20 : 7) als Laufmittel.

Ausbeute: 29.5 g (62,5 %); Fp (Mettler FP 61) : 125.1°C.

Analog Beispiel 3 wurde hergestellt:

<u>8-Methyl-6-(4-fluorphenyl)-2,3,6,7,8,9-hexahydro-1H-pyrido-/4,3-g/indol</u>

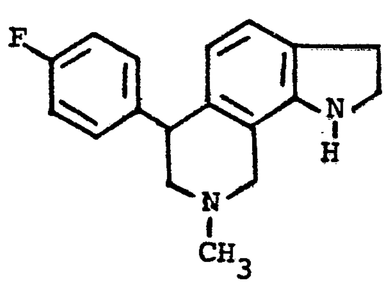

Das Rohprodukt wurde an Kieselgel (Kieselgel 60 der Fa. E. Merck, Darmstadt) mit einem Laufmittelgemisch aus Essigsäureethylester und MeOH (2 : 1) chromatografiert.

Ausbeute: 3.0 g (43 %); Fp (Mettler FP 61) : 137.6°C.

Herstellung des Hydrochlorids:

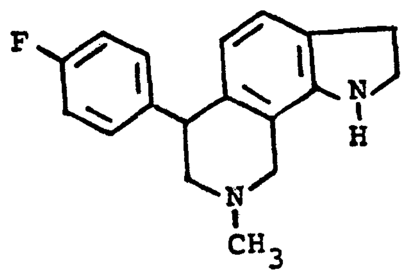

x 2HCl

3 b

Die Base wird mit etherischer Salzsäure in das Hydrochlorid überführt.

Fp (Mettler FP 61): 176.8°C.

Beispiel 4

1,8-Dimethyl-6-(fluorphenyl)-2,3,6,7,8,9-hexahydro-1H-pyrido/4,3-g7indol

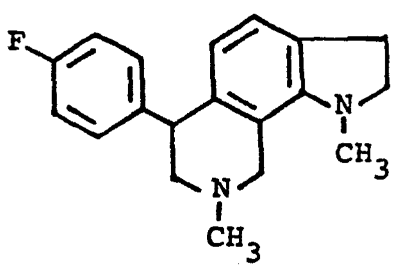

TP 67

2,3 g ( 0.00814 Mol) 8-Methyl-6-(fluorphenyl)-2,3,6,7,8,9-hexahydro-1H-pyrido/4,3-g/indol werden in 20 ml MeOH gelöst und unter Rühren bei Raumtemperatur mit 0.977 g (0.0163 Mol) Essigsäure und 0.791 ml (0.00976 Mol) Formalin versetzt. Das Reaktionsgemisch wird 1 Std. nachgerührt und anschließend werden in mehreren Portionen 1,53 g (0.0244 Mol) Natriumcyanoborhydrid zugegeben. Zur Vervollständigung der Reaktion rührt man 1.5 Std. bei Raumtemperatur nach. Dann zieht man das Lösungsmittel am Rotationsverdampfer ab, versetzt den Rückstand mit $H_2O$ und stellt mit 1 n NaOH alkalisch. Die wäßrige Phase wird mit $CH_2Cl_2$ extrahiert, über $Na_2SO_4$ getrocknet und das Lösungsmittel abrotiert. Den öligen Rückstand chromatografiert man an Kieselgel (Kieselgel 60 der Fa. E. Merck, Darmstadt) mit einem Laufmittelgemisch : $CH_2Cl_2$/MeOH (2.5 : 0.15). Das Produkt trocknet man unter Lichtausschluß im Exsikkator über $P_2O_5$ bei $10^{-2}$ bar.

Ausbeute: 1.8 g sirupöses Produkt.

Herstellung des Hydrochlorids:

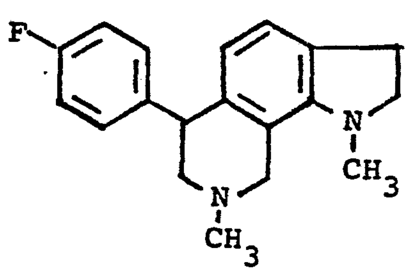

x 2HCl

4

Mit etherischer Salzsäure wird die Base ins Hydrochlorid überführt.

Fp (Mettler FP 61): 188.8°C (unter Zersetzung)

## Beispiel 5

1-(2-Pyrrolin-1-yl)-8-methyl-6-phenyl-2,3,6,7,8,9-hexahydro-1H-pyrido/4̄,3-g7indol

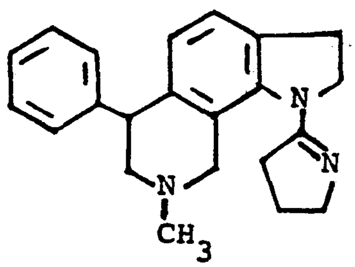

5

Zu einer Lösung von 3.4 g (0.04 Mol) Pyrrolidon-(2) in 8 ml Toluol wird unter Rühren und Kühlung bei einer Temperatur zwischen 2° und 0°C eine Lösung von 1.9 ml (0.02 Mol) Phosphoroxichlorid in 6 ml Toluol zugetropft. Man rührt 1 1/2 Std. bei Raumtemperatur und läßt über Nacht stehen. Man setzt anschließend eine Lösung von 5.2 g (0.02 Mol) 8-Methyl-6-phenyl-2,3,6,7,8,9-hexahydro-1H-pyrido/4̄,3-g7indol in 10 ml Toluol und 20 ml $CH_2Cl_2$ zu. $CH_2Cl_2$ wird abdestilliert und das Reaktionsgemisch unter Rühren 8 Stunden bei einer Temperatur zwischen 75° und 80°C gerührt. Der ausgefallene Feststoff wird abgesaugt und

zur Überführung in die freie Base mit 2 n NaOH und $H_2O$ versetzt. Die wäßrige Phase wird mit $CH_2Cl_2$ extrahiert, die $CH_2Cl_2$-Phase getrocknet und das Lösungsmittel entfernt. Den Rückstand chromatografiert man an Kieselgel 60) der Fa. E. Merck, Darmstadt) mit einem Laufmittelgemisch aus $CH_2Cl_2$/MeOH (2 : 1) und an Aluminiumoxid (Aluminiumoxid 90 der Fa. E. Merck, Darmstadt) mit einem Laufmittelgemisch aus $CH_2Cl_2$ und MeOH (20 : 0,25). Das Produkt wird im Exsikkator über $P_2O_5$ im Wasserstrahlvakuum getrocknet.

Ausbeute: 3.5 g (52,8 %); FP (Mettler FP 61): 57.9°C.

Beispiel 6

3-(1-Piperidinylmethyl)-6-phenyl-8-methyl-2,3,6,7,8,9-hexahydro-1H-pyrido/4,3-g/indol

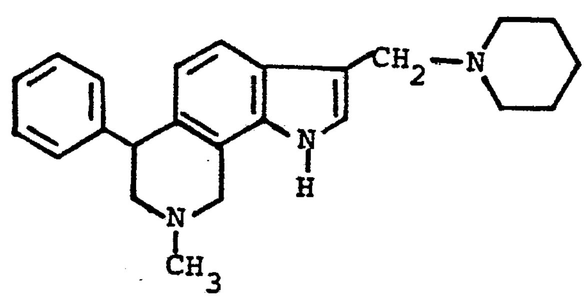

Eine eisgekühlte Lösung von 1.51 ml (0.01524 Mol) Piperidin und 1.14 ml $H_2O$ in 4.6 ml Essigsäure wird zusammen

mit 1.14 ml (0.014 Mol) Formalin auf einmal unter Rühren und Stickstoffspülung zu 2.0 g (0.00762 Mol) 8-Methyl-6-phenyl-6,7,8,9-tetrahydro-1H-pyrido/4,3-g/indol gegeben. Dann wird mit 2 n NaOH alkalisch gestellt und mit $CH_2Cl_2$ extrahiert. Die $CH_2Cl_2$-Phase wird über $Na_2SO_4$ getrocknet, und das Lösungsmittel wird am Rotationsverdampfer abgezogen. Den Rückstand chromatografiert man an Kieselgel (Kieselgel 60 der Fa. E. Merck, Darmstadt) mit einem Laufmittelgemisch aus $CH_2Cl_2/MeOH/N(C_2H_5)_3$ (15 : 6 : 0.5). Getrocknet wird das Produkt im Exsikkator über $P_2O_5$ im Wasserstrahlvakuum.

Ausbeute: 3.1 g (89.6 %); Fp (Mettler FP 61): 80,4°C.

Herstellung des Hydrochlorids

6 a

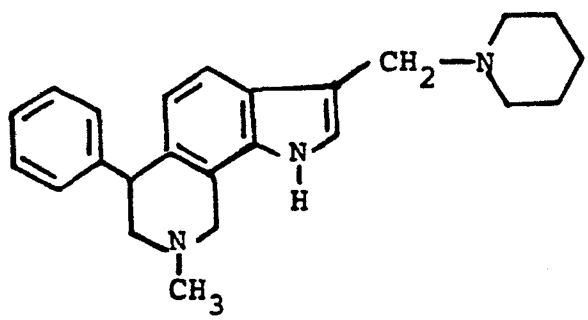

x 2HCl

Die Base wird in MeOH mit 1 n wäßriger HCl in das Hydrochlorid übergeführt. Nach Entfernen des Lösungsmittels wird im Exsikkator über NaOH im Wasserstrahlvakuum bei 95°C getrocknet.

Ausbeute: 3.3 g (91.5 %); Fp (Mettler FP 61): 212.8°C.

TP 67

Analog Beispiel 6 wurden hergestellt:

3-Dimethylaminomethyl-8-methyl-6-phenyl-6,7,8,9-tetrahydro-1H-pyrido/$\overline{4}$,3-g$\overline{7}$indol

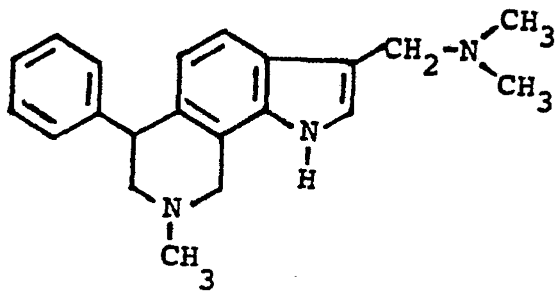

Ausbeute: 78 % d. Th.; Fp (Mettler FP 61): 80.9°C.

Herstellung des Hydrochlorids

6 b

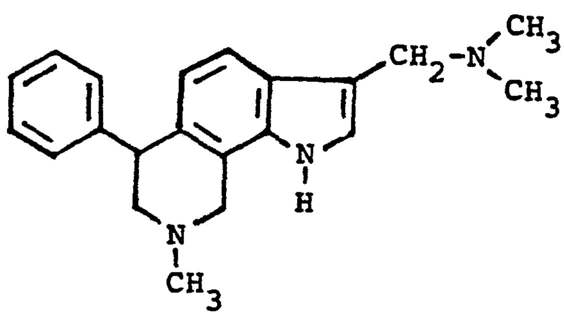

x 2HCl

Das Hydrochlorid wurde in MeOH mit 1 n wäßriger HCl erhalten. Fp (Mettler FP 61): 203°C.

TP 67

<u>3-(4-Morpholinylmethyl)-8-methyl-6-phenyl-6,7,8,9-tetrahydro-1H-pyrido/4̄,3-g7indol</u>

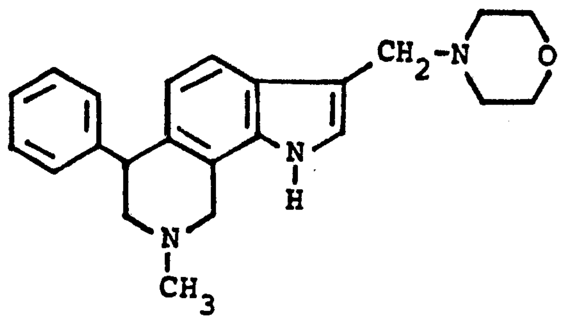

Das Rohprodukt wurde an Kieselgel (Kieselgel 60 der Fa. E. Merck, Darmstadt) mit einem Laufmittelgemisch $CH_2Cl_2$/ MeOH/$N(C_2H_2)_3$ (15 : 6 : 0.5) chromatografiert und die Substanz im Exsikkator getrocknet.

Ausbeute: 97.5 % d. Th.; Fp (Mettler FP 61): 77,3°C.

<u>Herstellung des Hydrochlorids</u>

<u>6 c</u>

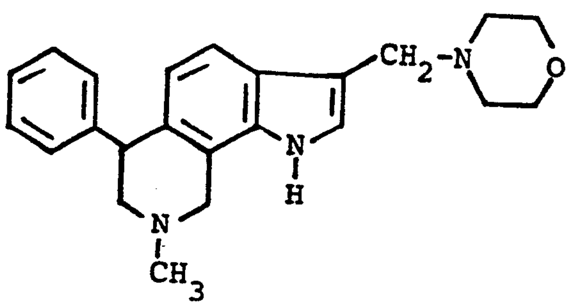

x 2 HCl

Das Hydrochlorid wurde in MeOH mit 1 n wäßriger HCl hergestellt. Fp (Mettler FP 61): 257°C.

<u>TP 67</u>

## Beispiel 7

**3-Methylaminoethyl-8-methyl-6-phenyl-6,7,8,9-tetrahydro-1H-pyrido[4,3-g]indol**

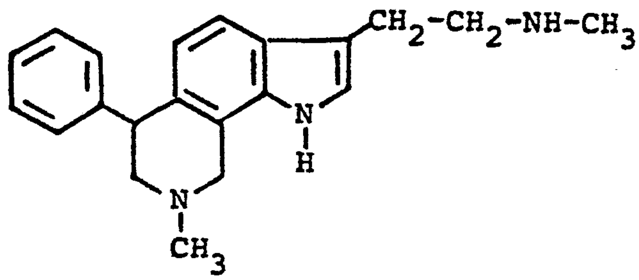

Man legt 4.9 g (0.129 Mol) $LiAlH_4$ in der 69.5 ml abs. THF vor, leitet Stickstoff über und tropft innerhalb von 15 Min. unter Rühren und Feuchtigkeitsausschluß bei Raumtemperatur eine Lösung von 11.3 g (0.0325 Mol) 3-(8-Methyl-6-phenyl-6,7,8,9-tetrahydro-1H-pyrido[4,3-g]indol-yl)-2-oxo-essigsäure-methylamid in 50 ml abs. THF zu. Anschließend erhitzt man 12 Std. am Rückfluß. Dann wird die Reaktionsmischung unter Kühlung und Rühren tropfenweise mit einer Lösung von 10 ml $H_2O$ in 40 ml THF versetzt. Den anorganischen Anteil saugt man ab und wäscht den Filterrückstand gut mit $CH_2Cl_2$. Anschließend engt man das Filtrat am Rotationsverdampfer ein und chromatografiert den Rückstand an Kieselgel (Kieselgel 60 der Fa. E. Merck, Darmstadt) über eine Säule mit einem Laufmittelgemisch aus $CH_2Cl_2/MeOH/N(C_2H_5)_3$ (15 : 10 : 0.5).

Ausbeute: 3.9 g (37 %).

Herstellung des Hydrochlorids

7 a

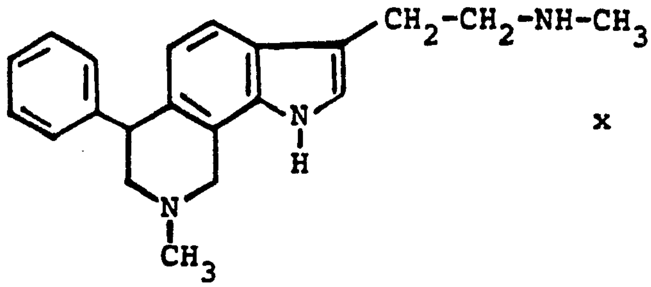

x 2HCl

Zur Herstellung des Hydrochlorids wird die Base in $CH_2Cl_2$ gelöst und die Lösung mit etherischer Salzsäure versetzt. Das Lösungsmittel wird abgezogen und der Rückstand mit Ether verrieben. Das HCl-Salz wird über NaOH bei 70°C im Wasserstrahlvakuum getrocknet.

Fp. (Mettler FP 61): 167.4°C.

Analog wurde hergestellt:

3-(Isopropylaminoethyl)-8-methyl-6-phenyl-6,7,8,9-tetrahydro-1H-pyrido/4,3-g/indol

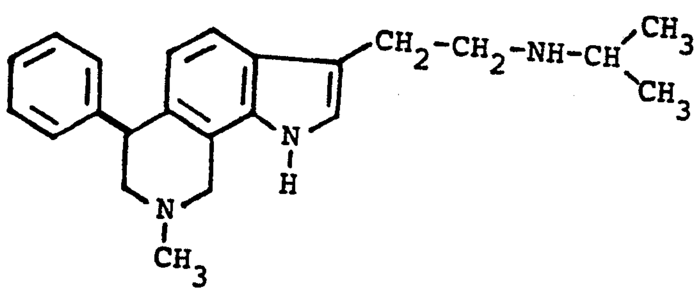

TP 67

Das Rohprodukt wurde an Kieselgel (Kieselgel 60 der Fa. E. Merck, Darmstadt) chromatografiert unter Verwendung eines Laufmittelgemisches: $CH_2Cl_2/MeOH/N(C_2H_5)_3$ (15 : 6 : 0,5). Anschließend wurde die Substanz im Exsikkator über $P_2O_5$ im Wasserstrahlvakuum getrocknet.

Ausbeute: 65 % d. Th.

<u>Herstellung des Hydrochlorids</u>

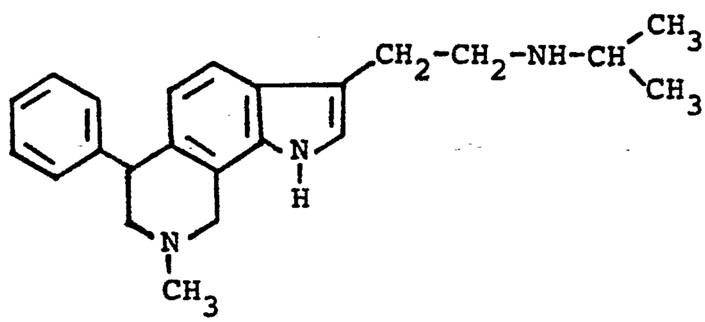
x 2 HCl

7 b

Das Hydrochlorid wurde wie vorstehend beschrieben erhalten. Fp (Mettler FP 61): 223.5°C.

Herstellung der Ausgangsverbindungen:

<u>3-(8-Methyl-6-phenyl-6,7,8,9-tetrahydro-1H-pyrido[4,3-g]indol-yl)-2-oxo-essigsäure-methylamid</u>

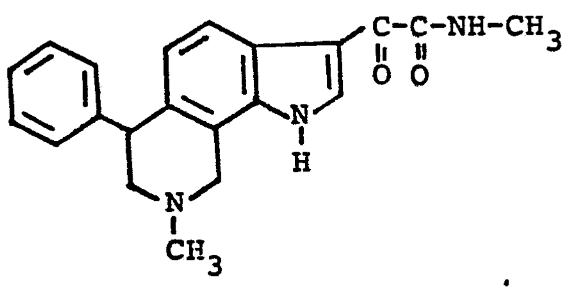

TP 67

Zu 5.5 g (0.0397 Mol) $K_2CO_3$ in 78.5 ml abs. Dioxan gibt man 7.92 ml (0.0918 Mol) Oxalylchlorid und kühlt unter Feuchtigkeitsausschluß auf 0°C. Nun wird unter Rühren innerhalb 30 Min. eine Lösung von 8-Methyl-6-phenyl-6,7,-8,9-tetrahydro-1H-pyrido[4,3-g]indol in 196 ml abs. Dioxan zugetropft und zur Vervollständigung der Reaktion 16 Std. bei Raumtemperatur nachgerührt. Anschließend fügt man 10,9 g (0.0791 Mol) $K_2CO_3$ zu und läßt dann eine Lösung von 12.1 g (0.389 Mol) wasserfreiem Methylamin in 100 ml abs. Dioxan zutropfen und 2 1/2 Std. nachreagieren. Das Lösungsmittel wird am Rotationsverdampfer abgezogen; der Rückstand wird mit 150 ml $H_2O$ versetzt und die wäßrige Phase mit 150 ml Diethylether ausgeschüttelt. Der unlösliche Substanzanteil wird abfiltriert, mit 50 ml $CH_2Cl_2$ gewaschen und über $P_2O_5$ bei 60°C im Wasserstrahlvakuum getrocknet.

Ausbeute: 10.1 g (74 %); Fp (Mettler FP 61): 224.7°C.

Auf analogem Wege wurde erhalten:

<u>3-(8-Methyl-6-phenyl-6,7,8,9-tetrahydro-1H-pyrido[4,3-g]indol-yl)-2-oxo-essigsäureisopropylamid</u>

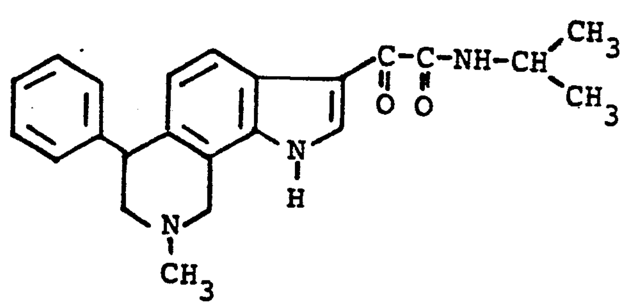

<u>TP 67</u>

Das Rohprodukt wurde mit einem Laufmittelgemisch aus Essigsäureethylester und MeOH (9 : 1) an Kieselgel (Kieselgel 60 der Fa. E. Merck, Darmstadt) chromatografiert. Anschließend wurde die Substanz über $P_2O_5$ im Wasserstrahlvakuum getrocknet.

Ausbeute: 65 % d. Th.

Herstellung des Hydrochlorids

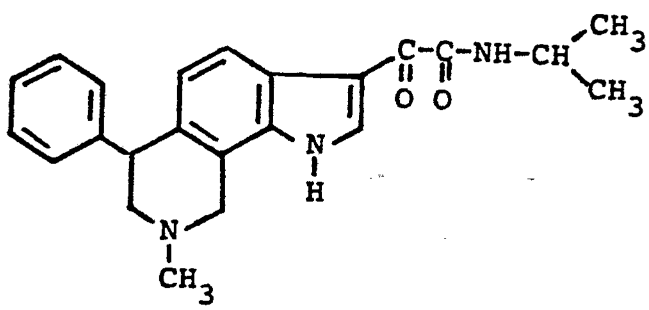

x HCl

Das Hydrochlorid wurde hergestellt wie für die Salze des Beispiels 7 beschrieben.

Fp (Mettler FP 61): 257.6°C.

Beispiel 8

3-(Isopropylaminoethyl)-8-methyl-6-phenyl-2,3,6,7,8,9-hexahydro-1H-pyrido/4,3-g/indol

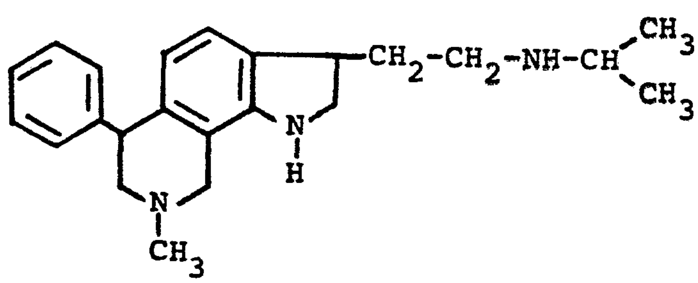

TP 67

2.1 g (0.00604 Mol) 3-(Isopropylaminoethyl)-8-methyl-6 phenyl-6,7,8,9-tetrahydro-1H-pyrido/4,3-g7indol werden in 25 ml (0.3275 Mol) Trifluoressigsäure unter Rühren und Feuchtigkeitsausschluß gelöst und anschließend bei einer Temperatur von 0° - +2°C tropfenweise mit einer Lösung von 1.76 g (0.0241 Mol) Trimethylaminoboran in 10 ml THF versetzt. Man rührt 1 Std. bei 0°C nach, gibt dann wieder eine Lösung von 1.76 g (0.0241 Mol) Trimethylaminoboran in 10 ml THF hinzu und rührt 2,5 Std. nach. Nun wird das Reaktionsgemisch mit 150 ml $H_2O$ versetzt, mit NaOH auf pH 13 eingestellt und das sich abscheidende ölige Produkt mit $CH_2Cl_2$ extrahiert. Die $CH_2Cl_2$-Phase wird mit $H_2O$ gewaschen, über $Na_2SO_4$ getrocknet und anschließend am Rotationsverdampfer vom Lösungsmittel befreit. Der Rückstand wird zweimal an Kieselgel (Kieselgel 60 der Fa. E. Merck, Darmstadt) chromatografiert. Fließmittel: zuerst $CH_2Cl_2/MeOH/N(C_2H_5)_3$ (15 : 4 : 0.25), dann Diethylether/$MeOH/N(C_2H_5)_3$ (15 : 3 : 0.25).

Ausbeute: 1.3 g farblose sirupöse Substanz (61.5 %).

Herstellung des Hydrochlorids

8

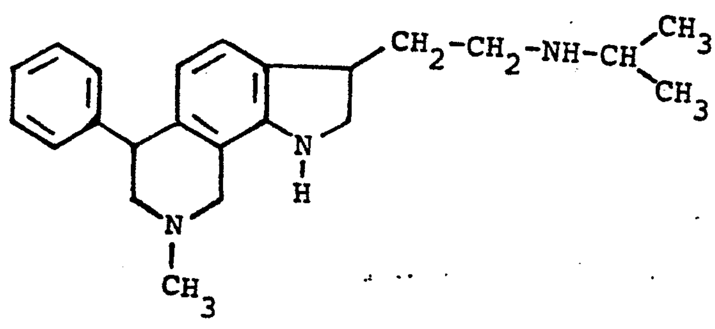

x 3HCl

TP 67

Das Hydrochlorid wurde erhalten wie im Beispiel 7 beschrieben.
Fp (Mettler FP 61): 223°C.

Beispiel 9

3,7-Dimethyl-9-phenyl-1,2,3,4,6,7,8,9-octahydro-5H-dipyrido/4̄,3-g:3',4'-b7indol

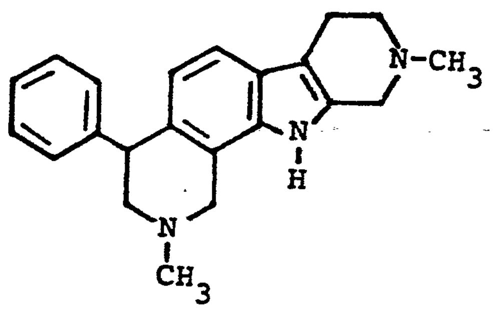

0.8 g ( 0.0025 Mol) 3-(Methylaminoethyl)-8-methyl-6-phenyl-6,7,8,9-tetrahydro-1H-pyrido/4̄,3-g7indol, 0.23 ml (0.0028 Mol) 40 % Formalinlösung und 1.5 ml 0,1 n HCl (0.00015 Mol) werden mit 0.15 ml 1 n HCl (0.00015 Mol) versetzt und anschließend 2 Std. bei Raumtemperatur gerührt. Die sich bildende kristalline Substanz wird mit 2 ml Ethylalkohol in Lösung gebracht und über Nacht stehengelassen. Das Lösungsmittel zieht man am Rotationsverdampfer ab, versetzt den Rückstand mit wäßriger $KHCO_3$-Lösung und extrahiert die wäßrige Phase mit $CH_2Cl_2$. Die $CH_2Cl_2$-Phase wird über $Na_2SO_4$ getrocknet und das Lösungsmittel am Rotationsverdampfer abgezogen. Der Rückstand wird an Kieselgel(Kieselgel 60 der Fa. E. Merck, Darmstadt) über eine

Säule mit Laufmittel Essigester/MeOH/ $N(C_2H_5)_3$ (9 : 3.5 : 0.25) getrennt.

Ausbeute: 0.6 g (73 %).

Herstellung des Hydrochlorids

9

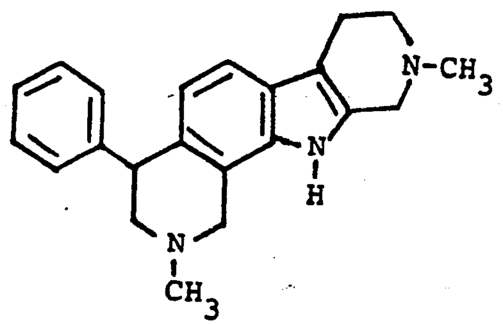

x 2HCl

Das Hydrochlorid der Base wurde in Analogie zu Beispiel 7 erhalten.
Fp (Micro-Kofler): 236° - 237°C.

Beispiel 10

2-(N-Piperidino)-3-ethylendioxi-6-phenyl-8-methyl-6,7,8,9-tetrahydro-3H-pyrido[4,3-g]indol

10 a

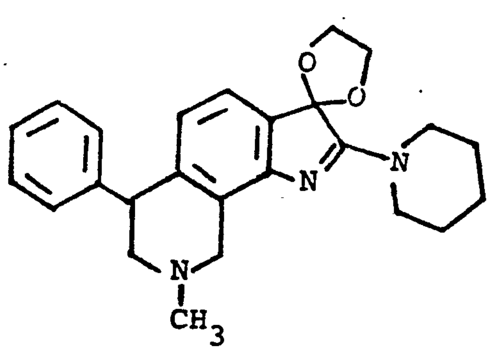

TP 67

3.1 g (0.00879 Mol) 2-Thiono-3-ethylendioxi-6-phenyl-8-methyl-2,3,6,7,8,9-hexahydro-1H-pyrido/4,3-g7indol werden unter Rühren in 42.5 ml (0.429 Mol) Piperidin gelöst und unter Feuchtigkeitsausschluß und Stickstoffspülung 1 Std. auf 110°C erwärmt. Das überschüssige Piperidin wird am Rotationsverdampfer abdestilliert, der Rückstand in $CH_2Cl_2$ gelöst und die $CH_2Cl_2$-Phase 3 mal mit $H_2O$ ausgezogen. Man trocknet die organischen Phase über $Na_2SO_4$ und zieht das Lösungsmittel am Rotationsverdampfer ab. Den Rückstand chromatografiert man an Aluminiumoxid (Aluminiumoxid 90 der Fa. E. Merck, Darmstadt) mit einem Laufmittelgemisch aus Dichlormethan/ Methanol (20 : 0.05). Getrocknet wird die Substanz über $P_2O_5$ im Wasserstrahlvakuum.

Ausbeute: 1.1 g (24.5 %); Fp (Micro-Kofler): 215° - 216°C.

Analog Beispiel 10 wurde erhalten:

<u>2-Butylimino-3-ethylendioxi-6-phenyl-8-methyl-2,3,6,7,8,9-hexahydro-1H-pyrido/4,3-g7indol</u>

10 b

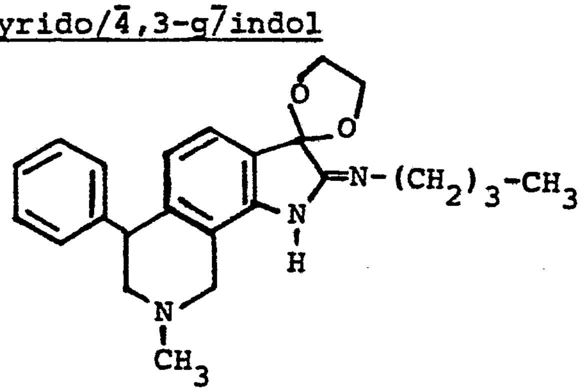

Das Rohprodukt wurde an Kieselgel (Kieselgel 60 der Fa.

E. Merck, Darmstadt) chromatografiert mit einem Laufmittelgemisch aus Essigsäureethylester und Methanol ( 9 : 1). Die Reinsubstanz wurde über $P_2O_5$ bei 60°C im Wasserstrahlvakuum getrocknet.

Ausbeute: 49 % d. Th; Fp (Mettler FP 61): 87,3°C.

Herstellung der Ausgangsstoffe:

<u>2-Thiono-3-ethylendioxi-6-phenyl-8-methyl-2,3,6,7,8,9-hexahydro-1H-pyrido-/4̄,3-g/indol</u>

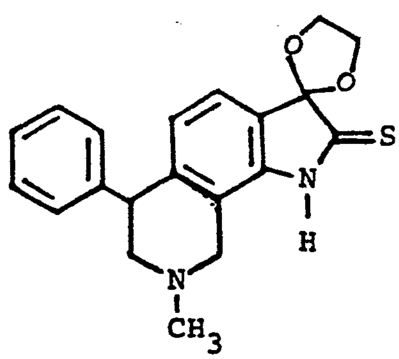

Eine Aufschlämmung von 1.68 g (0.005 Mol) 2-Oxo-3-ethylendioxi-6-phenyl-8-methyl-2,3,6,7,8,9-hexahydro-1H-pyrido-/4̄,3-g/indol und 1.0 g (0.0025 Mol) Lawesson-Reagenz in 10 ml abs. Toluol wird unter Feuchtigkeitsausschluß und Rühren 1 Std. am Rückfluß erhitzt. Die Reaktionsmischung befreit man anschließend am Rotationsverdampfer vom Lösungsmittel und chromatografiert den Rückstand an Kieselgel (Kieselgel 60 der Fa. E. Merck, Darmstadt) mit einem

Laufmittelgemisch aus $CH_2Cl_2$ und MeOH (20 : 1). Getrocknet wird die Substanz über $P_2O_5$ im Wasserstrahlvakuum.

Ausbeute: 0.85 g (49 %).

2-Oxo-3-ethylendioxi-6-phenyl-8-methyl-2,3,6,7,8,9-hexahydro-1H-pyrido/4,3-g/indol

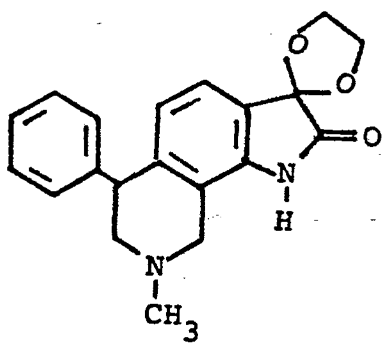

20.6 g (0.0624 Mol) 8-Methyl-6-phenyl-6,7,8,9-tetrahydro-1H-pyrido/4,3-g/indol-2,3-dion, 21 ml (0.374 Mol) Ethylenglycol und 0.336 g (0.00176 Mol) Toluolsulfonsäure-(4)-monohydrat werden unter Rühren mit 100 ml Dichlorethan versetzt und 3.5 Std. am Wasserabscheider unter Rückfluß erhitzt. Die Reaktionsmischung wird mit Eis versetzt und mit wäßriger $KHCO_3$-Lösung auf pH 6.8 eingestellt. Man extrahiert mit $CH_2Cl_2$, wäscht die $CH_2Cl_2$-Phase mit $H_2O$, trocknet über $Na_2SO_4$ und zieht das Lösungsmittel im Rotationsverdampfer ab. Den Rückstand chromatografiert man an $Al_2O_3$ (Aluminiumoxid der Fa. E. Merck, Darmstadt) mit einem Laufmittelgemisch aus Essigsäureethylester und MeOH (20 : 0.2). Die Substanz wird nach Verreiben mit

Essigsäureethylester / Petrolether kristallin. Sie wird über $P_2O_5$ bei 80°C im Vakuum getrocknet.

Ausbeute: 12.5 g (59.5 %);

Fp (Mettler FP 61): 221.2°C.

TP 67

Patentansprüche

1. Pyridoindole der allgemeinen Formel I

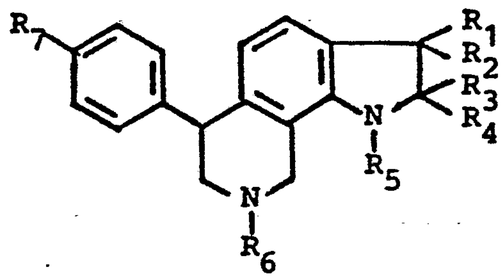

I

in welcher

$R_1$ für Wasserstoff oder $C_1$-$C_4$-Alkyl steht, welches gegebenenfalls durch den Rest 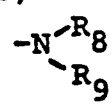 substituiert ist,

$R_2$ und $R_3$ für H stehen oder eine Bindung bilden, oder

$R_1$ und $R_2$ zusammen für O, $-O-CH_2-CH_2O-$, $-S-CH_2-CH_2-S-$ stehen,

$R_4$ für H oder den 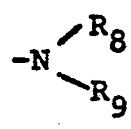 -Rest steht oder

$R_3$ und $R_4$ für O stehen, oder

$R_1$ und $R_4$ Glieder eines N-haltigen Sechsrings sind und

$R_8$ und $R_9$ für H oder $C_1$-$C_4$-Alkyl stehen oder gegebenenfalls mit dem N-Atom einen heterocyclischen 5- oder 6-Ring bilden, der gegebenenfalls auch ein weiteres Heteroatom aus der Reihe N, O oder S enthalten kann,

$R_5$ für H, $C_1$-$C_4$-Alkyl oder die Gruppe 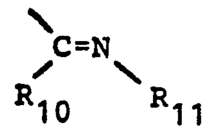 steht, oder

$R_5$ und $R_3$ eine Bindung bilden, und

$R_{10}$ und $R_{11}$ für $C_1$-$C_4$-Alkyl stehen oder Glieder eines N-haltigen 5- oder 6-Rings sind,

$R_6$ für H oder $C_1$-$C_4$-Alkyl steht und

$R_7$ für Halogen, insbesondere für F, Cl oder Br steht

sowie deren Säuredditionssalze.

2. Verbindungen der allgemeinen Formel I, gemäß Anspruch 1, in der

$R_1$ für H oder $C_1$-$C_3$-Alkyl steht, das durch den Rest 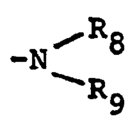 substituiert ist, wobei $R_8$ und $R_9$ unabhängig voneinander für H oder

$C_1$-$C_4$-Alkyl stehen oder zusammen mit dem N-Atom einen Piperidin-, Pyrrolidin-, Morpholin- oder Piperazinring bilden,

$R_4$,$R_5$ für H stehen,

$R_2$ und $R_3$ für H stehen oder eine Bindung bilden,

$R_6$ für $CH_3$ und

$R_7$ für H oder F steht

sowie deren Säureadditionssalze.

3. Verbindungen der allgemeinen Formel I, gemäß Anspruch 1, in der

$R_1$, $R_4$ und $R_5$ für H stehen,

$R_2$ und $R_3$ für H stehen oder eine Bindung bilden,

$R_6$ für $CH_3$ und

$R_7$ für F steht.

4. Arzneimittel enthaltend mindestens eine Verbindung der allgemeinen Formel I gemäß Anspruch 1.

5. Verwendung von Verbindungen der allgemeinen Formel I gemäß Anspruch 1 bei der Bekämpfung von Krankheiten.

6. Verwendung von Verbindungen der allgemeinen Formel I gemäß Anspruch 1 zur Beeinflussung des zentralen Nervensystems.

7. Verwendung von Verbindungen der allgemeinen Formel I gemäß Anspruch 1 als Anxiolytika, Nootropika oder vorzugsweise als Antidepressiva.

8. Verfahren zur Herstellung von Verbindungen der allgemeinen Formel I gemäß Anspruch 1, dadurch gekennzeichnet, daß man

a) Verbindungen der allgmeinen Formel II

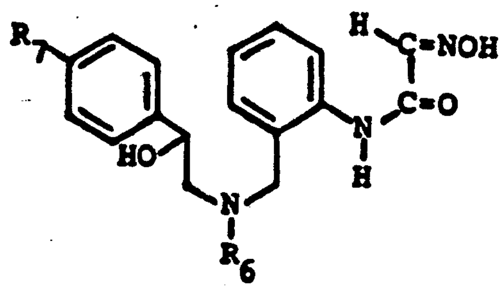

II

in der

$R_6$ und $R_7$ die in den Ansprüchen 1 bis 3 angegebene Bedeutung haben,

einer doppelten Ringschlußreaktion in Gegenwart eines wasserabspaltenden Mittels unterwirft oder

b) Verbindungen der allgemeinen Formel III

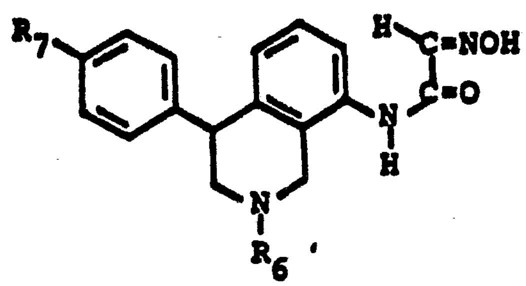

III

in der

$R_6$ und $R_7$ die in den Ansprüchen 1 bis 3 angegebene Bedeutung besitzen,

in Gegenwart eines wasserabspaltenden Mittels cyclisiert

und die nach a) oder b) erhaltenen Isatine anschliessend stufenweise reduziert.

9. Verbindungen der allgemeinen Formel II,

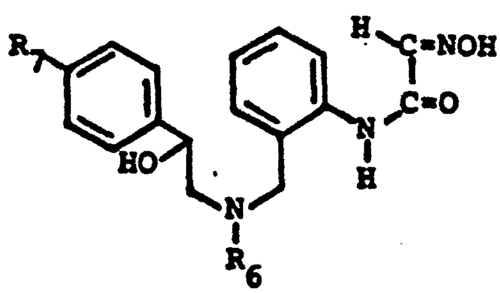

II

in welcher

$R_6$ für Wasserstoff oder $C_1$-$C_4$-Alkyl und

$R_7$ für Wasserstoff oder Halogen, insbesondere für F, Cl oder Br steht.

10. Verbindungen der allgemeinen Formel III,

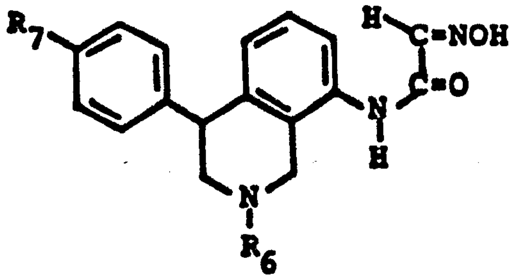

III

in welcher

$R_6$ für Wasserstoff oder $C_1$-$C_4$-Alkyl und

$R_7$ für Wasserstoff oder Halogen, insbesondere für F, Cl oder Br steht.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0140070
Nummer der Anmeldung

EP 84110732.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | CHEMICAL ABSTRACTS, Band 86, Nr. 25, 20. Juni 1977, Columbus, Ohio, USA<br>R. TACHIKAWA "Pyridoindole derivatives"<br>Seite 611, Spalte 2, Abstract-Nr. 189 902k<br>& Japan. Kokai 76, 118,799, 18. Oktober 1976<br>-- | 1,4 | C 07 D 471/04<br>C 07 D 491/20<br>C 07 D 495/20<br>C 07 D 217/02<br>C 07 D 217/04<br>C 07 D 217/14<br>C 07 C 131/00<br>A 61 K 31/47 |
| A | CHEMICAL ABSTRACTS, Band 84, Nr. 11, 15. März 1976, Columbus, Ohio, USA<br>S. NARUTO "Bischler-Napieralski reactions of N-(2-indolylethyl)-acetamide"<br>Seite 440, Spalte 1, Abstract-Nr. 74 140b<br>& Chem. Pharm. Bull. 1975, 23(12), 3184-8<br>-- | 1 | |

RECHERCHIERTE SACHGEBIETE (Int. Cl. 3)

C 07 D 471/00
C 07 D 491/00
C 07 D 495/00
C 07 D 217/00
C 07 C 131/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-4, 8-10

Unvollständig recherchierte Patentansprüche: –

Nicht recherchierte Patentansprüche: 5-7

Grund für die Beschränkung der Recherche:

Artikel 52(4) EPÜ: Verfahren zur therapeutischen Behandlung des menschlichen oder tierischen Körpers

| Recherchenort | Abschlußdatum der Recherche | Prüfer |
|---|---|---|
| WIEN | 07-12-1984 | ONDER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

0140070

Nummer der Anmeldung

Europäisches Patentamt

# EUROPÄISCHER TEILRECHERCHENBERICHT

EP 84110732.9

| EINSCHLÄGIGE DOKUMENTE | | | KLASSIFIKATION DER ANMELDUNG (Int. Cl.4) |
| --- | --- | --- | --- |
| Kategorie | Kennzeichnung des Dokuments mit Angabe, soweit erforderlich, der maßgeblichen Teile | betrifft Anspruch | |
| A | DE - B2 - 2 424 372 (BASF)<br>* Patentanspruch *<br>-- | 9,10 | |
| A,D | DE - B2 - 1 670 694 (HOECHST)<br>* Formel I *<br>-- | 10 | |
| A | R.C. ELDERFIELD "Heterocyclic Compounds", Band 3, 1952<br>JOHN WILEY & SONS, New York<br>Seiten 208, 209<br>---- | 8 | |
| | | | RECHERCHIERTE SACHGEBIETE (Int. Cl.4) |